(19) Europäisches Patentamt
European Patent Office
Office européen des brevets

(11) **EP 4 810 525 A1**

(12) **EUROPEAN PATENT APPLICATION**
published in accordance with Art. 153(4) EPC

(43) Date of publication:
**23.09.2026 Bulletin 2026/39**

(21) Application number: **24891478.0**

(22) Date of filing: **14.11.2024**

(51) International Patent Classification (IPC):
***C07K 19/00*** (2006.01) ***A61K 35/17*** (2025.01)
***A61P 35/00*** (2006.01) ***C07K 14/705*** (2006.01)
***C07K 16/30*** (2006.01) ***C12N 5/10*** (2006.01)
***C12N 5/0783*** (2010.01) ***C12N 15/12*** (2006.01)
***C12N 15/13*** (2006.01) ***C12N 15/62*** (2006.01)

(52) Cooperative Patent Classification (CPC):
**A61K 35/17; A61P 35/00; C07K 14/435; C07K 14/705; C07K 16/00; C07K 16/30; C07K 19/00; C12N 5/06; C12N 5/10; C12N 15/62**

(86) International application number:
**PCT/JP2024/040523**

(87) International publication number:
**WO 2025/105442 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.11.2023 JP 2023194184**

(71) Applicant: **The University of Osaka**
**Suita-shi, Osaka 565-0871 (JP)**

(72) Inventors:
- **OKAIRI, Yuzuru**
  **Osaka-shi, Osaka 540-0021 (JP)**
- **SONE, Masayuki**
  **Osaka-shi, Osaka 540-0021 (JP)**
- **KUWANO, Nozomi**
  **Osaka-shi, Osaka 540-0021 (JP)**
- **AKAMINE, Hiroki**
  **Osaka-shi, Osaka 540-0021 (JP)**
- **URA, Hasumi**
  **Osaka-shi, Osaka 540-0021 (JP)**
- **TANAKA, Rina**
  **Osaka-shi, Osaka 540-0021 (JP)**
- **OKUMURA, Yuto**
  **Osaka-shi, Osaka 540-0021 (JP)**
- **MASUMURA, Ryo**
  **Osaka-shi, Osaka 540-0021 (JP)**
- **HOSEN, Naoki**
  **Suita-shi, Osaka 565-0871 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) CHIMERIC ANTIGEN RECEPTOR

(57) Provided is a novel chimeric antigen receptor. The chimeric antigen receptor contains an scFv region having a structure in which the C-terminus of a heavy-chain variable region and the N-terminus of a light-chain variable region are linked via a linker, wherein the heavy-chain variable region contains a heavy-chain CDR1 having the amino acid sequence of SEQ ID NO: 1, a heavy-chain CDR2 having the amino acid sequence of SEQ ID NO: 2, and/or a heavy-chain CDR3 having the amino acid sequence of SEQ ID NO: 3; and/or the light-chain variable region contains a light-chain CDR1 having the amino acid sequence of SEQ ID NO: 5, a light-chain CDR2 having the amino acid sequence of SEQ ID NO: 6, and/or a light-chain CDR3 having the amino acid sequence of SEQ ID NO: 7; and the number of amino acid residues constituting the linker is 15 to 50.

EP 4 810 525 A1

Fig. 1

LTR
VH
linker
VL
CD28
Hinge/Spacer
CD28
transmembrane
CD28-CD3ζ
Signal domain
LTR
CD98 scFv
LTR
VL
linker
VH
CD28
Hinge/Spacer
CD28
transmembrane
CD28-CD3ζ
Signal domain
LTR

## Description

Technical Field

A novel chimeric antigen receptor and use of the chimeric antigen receptor are disclosed.

Background Art

The present inventors identified R8H283 antibody, which specifically binds to myeloma cells in the bone marrow of human multiple-myeloma patients (PTL 1). The R8H283 antibody was obtained as an antibody that recognizes the human CD98hc heavy chain (CD98hc). Subsequently, the R8H283 antibody was confirmed to recognize an epitope that contains an N-glycosylation site of human CD98hc, and that is exposed by inhibiting N-linked glycosylation.

Although several antibodies against CD98 are known, none is currently on the market. Additionally, there is currently limited information available about its specific use as CAR-T cells.

Citation List

Patent Literature

PTL 1: WO2017/026497A

Non-patent Literature

NPL 1: J Immunol. 2011 Feb 1; 186(3): 1840-1848
NPL 2: J Immunol. 2009 Nov 1; 183(9): 5563-74.
NPL 3: N Engl J Med. 2014 Oct 16; 371(16): 1507-17.
NPL 4: Nat Med. 2017 23, 1436-1443.

Summary of Invention

Technical Problem

An object is to provide a novel chimeric antigen receptor. Another object is to provide a more effective means for treating diseases such as malignant tumors.

Solution to Problem

CAR-T cells containing a single-chain variable fragment (scFv) derived from the R8H283 antibody were produced. As shown in the Examples described below, CAR-T cells containing an scFv in which the light-chain variable region (VL) and heavy-chain variable region (VH) were arranged in this order from the N-terminus showed limited proliferation and also showed a limited antitumor effect in vivo. On the other hand, CAR-T cells containing an scFv with VH and VL arranged in this order from the N-terminus were shown to have excellent proliferation and excellent in vivo antitumor effects. Further research and study were conducted based on the findings and led to the present invention, including the following subject matter

Item 1.

A chimeric antigen receptor comprising an scFv region having a structure in which the C-terminus of a heavy-chain variable region and the N-terminus of a light-chain variable region are linked via a linker,
wherein

the heavy-chain variable region contains

a heavy-chain CDR1 having the amino acid sequence of SEQ ID NO: 1,
a heavy-chain CDR2 having the amino acid sequence of SEQ ID NO: 2, and/or
a heavy-chain CDR3 having the amino acid sequence of SEQ ID NO: 3, and/or

the light-chain variable region contains

a light-chain CDR1 having the amino acid sequence of SEQ ID NO: 5,
a light-chain CDR2 having the amino acid sequence of SEQ ID NO: 6, and/or
a light-chain CDR3 having the amino acid sequence of SEQ ID NO: 7; and

the number of amino acid residues constituting the linker is 15 to 50.

Item 2.

The chimeric antigen receptor according to Item 1, wherein

the amino acid sequence of a heavy-chain FR region of the heavy-chain variable region contains an amino acid sequence having at least 80% identity to the amino acid sequence of a heavy-chain FR region of the amino acid sequence of SEQ ID NO: 4, and/or
the amino acid sequence of a light-chain FR region of the light-chain variable region contains an amino acid sequence having at least 80% identity to the amino acid sequence of a light-chain FR region of the amino acid sequence of SEQ ID NO: 8.

Item 3.

A chimeric antigen receptor, comprising

a heavy-chain variable region,
a light-chain variable region, and
a linker,

wherein

the amino acid sequence of the heavy-chain variable region contains an amino acid sequence having at least 90% identity to the amino acid sequence of SEQ ID NO: 4, and/or
the amino acid sequence of the light-chain variable region contains an amino acid sequence having at least 90% identity to the amino acid sequence of SEQ ID NO: 8; and
the number of amino acid residues constituting the linker is 15 to 50.

Item 4.

The chimeric antigen receptor according to any one of Items 1 and 3, having a structure in which the heavy-chain variable region, the linker, the light-chain variable region, a hinge sequence, a transmembrane domain, and an intracellular signaling domain are arranged in this order from the N-terminus.

Item 5.

A polynucleotide encoding the chimeric antigen receptor of any one of Items 1 to 4.

Item 6.

A cell comprising the polynucleotide of Item 5.

Item 7.

The cell according to Item 6, which is a chimeric antigen receptor T cell.

Item 8.

A pharmaceutical composition comprising the cell of Item 6 or 7.

Item 9.

The pharmaceutical composition according to Item 8, which is for use in the treatment and/or prevention of cancer.

Item 10.

A method for producing a chimeric antigen receptor T cell, comprising culturing a T cell transfected with a polynucleotide encoding the chimeric antigen receptor of any one of Items 1 to 4 in the presence of dasatinib.

Item 11.

A chimeric antigen receptor T cell produced according to the method of Item 10.

Item 12.

A pharmaceutical composition comprising the cell of Item 11.

Item 13.

The pharmaceutical composition according to Item 12, which is for use in the treatment and/or prevention of cancer.

Advantageous Effects of Invention

A novel chimeric antigen receptor and a cell containing a polynucleotide encoding the receptor are provided. Use of the chimeric antigen receptor enables effective treatment or prevention of diseases such as malignant tumors.

Brief Description of Drawings

Fig. 1 shows a schematic diagram of a prepared chimeric antigen receptor construct.
Fig. 2 shows the results of evaluating the proliferation of CAR-T cells.
Fig. 3 shows the results of evaluating the cytotoxic activity (luminescence value) of CAR-T cells.
Fig. 4 shows the results of evaluating the IFN-γ production ability of CAR-T cells.
Fig. 5 shows the results of evaluating the antitumor effect (luminescence value) of CAR-T cells.
Fig. 6 shows the results of evaluating the proliferation of CAR-T cells.
Fig. 7 shows the results of evaluating the cytotoxic activity (luminescence value) of CAR-T cells.
Fig. 8 shows the results of evaluating the IFN-γ production ability of CAR-T cells.
Fig. 9 shows the results of evaluating the change in CAR-T cell count upon continuous antigen stimulation.
Fig. 10 shows the results of evaluating the cytotoxic activity (luminescence value) of CAR-T cells upon continuous antigen stimulation.
Fig. 11 shows the results of evaluating the antitumor effect (luminescence value) of CAR-T cells.
Fig. 12 shows the results of evaluating the cytotoxic activity (luminescence value) of CAR-T cells.
Fig. 13 shows the results of evaluating the IFN-γ production ability of CAR-T cells.
Fig. 14 shows the results of evaluating the antitumor effect (luminescence value) of CAR-T cells.
Fig. 15 shows the results of evaluating the antitumor effect (luminescence value) of CAR-T cells.
Fig. 16 shows the results of evaluating the aggregation scores of Fv structures.
Fig. 17 shows the results of evaluating the cytotoxic activity (luminescence value) of CAR-T cells.
Fig. 18 shows the results of evaluating the IFN-γ production ability of CAR-T cells.
Fig. 19 shows the results of evaluating the antitumor effect (luminescence value) of CAR-T cells.
Fig. 20 shows the results of evaluating the antitumor effect (luminescence value) of CAR-T cells.
Fig. 21 shows the results of evaluating the cytotoxic activity (luminescence value) of CAR-T cells.
Fig. 22 shows the results of evaluating the IFN-γ production ability of CAR-T cells.
Fig. 23 shows the results of evaluating the antitumor effect (luminescence value) of CAR-T cells.
Fig. 24 shows the evaluation schedule for the antitumor effect (luminescence value) of CAR-T cells.
Fig. 25 shows the results of evaluating the antitumor effect (luminescence value) of CAR-T cells.

Fig. 26 shows the results of evaluating the antitumor effect (luminescence value) of CAR-T cells.
Fig. 27 shows the results of evaluating the antitumor effect (luminescence value) of CAR-T cells.
Fig. 28 shows the results of evaluating the cytotoxic activity (luminescence value) of CAR-T cells.
Fig. 29 shows the results of evaluating the cytotoxic activity (luminescence value) of CAR-T cells.
Fig. 30 shows the results of evaluating the cytotoxic activity (luminescence value) of CAR-T cells.
Fig. 31 shows the results of evaluating the cytotoxic activity (luminescence value) of CAR-T cells.
Fig. 32 shows the results of evaluating the cytotoxic activity (luminescence value) of CAR-T cells.
Fig. 33 shows the results of evaluating the cytotoxic activity (luminescence value) of CAR-T cells.
Fig. 34 shows the results of evaluating the IFN-γ production ability of CAR-T cells.
Fig. 35 shows the results of evaluating the IFN-γ production ability of CAR-T cells.
Fig. 36 shows the results of evaluating the IFN-γ production ability of CAR-T cells.
Fig. 37 shows the results of evaluating the IFN-γ production ability of CAR-T cells.
Fig. 38 shows the results of evaluating the IFN-γ production ability of CAR-T cells.
Fig. 39 shows the results of evaluating the IFN-γ production ability of CAR-T cells.
Fig. 40 shows the results of evaluating the antitumor effect (luminescence value) of CAR-T cells.
Fig. 41 shows the results of evaluating the antitumor effect (luminescence value) of CAR-T cells.
Fig. 42 shows the results of evaluating the antitumor effect (luminescence value) of CAR-T cells.
Fig. 43 shows the results of evaluating the antitumor effect (luminescence value) of CAR-T cells.
Fig. 44 shows the results of evaluating the antitumor effect (luminescence value) of CAR-T cells.
Fig. 45 shows the results of evaluating the cytotoxic activity (luminescence value) of CAR-T cells.
Fig. 46 shows the results of evaluating the IFN-γ production ability of CAR-T cells.
Fig. 47 shows the results of evaluating the antitumor effect (luminescence value) of CAR-T cells.

## Description of Embodiments

### 1. Chimeric Antigen Receptor (CAR)

A "chimeric antigen receptor" generally refers to an artificial T-cell receptor (TCR)-like protein and is known as a protein constructed so as to have the antigen recognition site (corresponding to the extracellular domain) expressed on the cell membrane of a T cell replaced with a desired antigen recognition site and to allow the T cell itself to more effectively exert its functionality, such as cytotoxic activity. The chimeric antigen receptor typically has its single-chain antibody (scFv) composed of a light-chain variable region bound to a heavy-chain variable region in tandem on the N-terminus side and its T-cell receptor (TCR) ζ chain on the C-terminus side. A cell such as a T cell expressing a chimeric antigen receptor recognizes an antigen in its scFv region, and then intracellularly transfers a recognition signal through the ζ chain.

In an embodiment, the chimeric antigen receptor preferably has a structure in which the following are arranged from its N-terminus in order: an scFv region, a hinge or spacer sequence, a transmembrane domain, and an intracellular signaling domain.

In an embodiment, the chimeric antigen receptor preferably contains an scFv region having a structure in which the C-terminus of the heavy-chain variable region and the N-terminus of the light-chain variable region are linked via a linker.

The number of amino acid residues constituting the linker is preferably 15 to 50. The lower limit of the range may be 16, 17, or 18, and the upper limit may be 45, 40, 35, 30, 25, 24, 23, or 22.

The linker can be of any type, and examples include a linker composed of glycine and serine, such as a G4S linker (GGGGS: SEQ ID NO: 22), and a 218 linker (GSTSGSGKPGSGEGSTKG; SEQ ID NO: 21). The linker may have a structure in which these linkers are repeated. The linker sequence may be the same as or different from the hinge or spacer sequence provided between the scFv region and the transmembrane domain.

CD98 (or "4F2") is a heterodimer protein of about 120 kDa that is expressed on a cell membrane and is known to function as an amino acid transporter. CD98hc is a type II transmembrane protein of about 80 kDa that constitutes CD98 and may be referred to as "4F2hc" or "SLC3A2." Although there are several isoforms (e.g., b, c, e, and f) for human CD98hc, these isoforms have a common amino acid sequence for their extracellular domain. The sequence list shows the amino acid sequence of isoform f, which is a typical human CD98hc, as SEQ ID NO: 17. Human CD98hc has a structure in which N-glycans can bind to the position 264, position 280, position 323, and position 405 of the amino acid sequence of SEQ ID NO: 17. Although the amino acid sequence of isoform f of human CD98hc is referenced as a typical example in this specification, a person skilled in the art would understand that the corresponding sequence and the corresponding amino acid residues are present in other isoforms and function equivalently.

N-glycans on CD98hc in myeloma cells, myeloma progenitor cells, and other tumor cells are considered to have undergone a change (e.g., failure of N-linked glycosylation). This is probably because myeloma cells, myeloma progenitor cells, and other tumor cells are always subjected to endoplasmic reticulum stress, and N-linked glycosylation is being

inhibited. This suggests that the amino acid sequence of CD98hc, which an antibody and the like cannot access in other normal cells, is exposed on the surface of myeloma cells, myeloma progenitor cells, or other tumor cells, and so the antibody and the like can access the amino acid sequence. In this specification, this region of CD98hc specific to the myeloma cells, myeloma progenitor cells, and other tumor cells may be referred to as the "region that contains an N-glycosylation site of human CD98hc, and that is exposed by inhibiting N-linked glycosylation" or the "region affected by the presence of an N-glycan." Because an epitope is present in this region, the antibody can specifically recognize a myeloma cell, myeloma progenitor cell, or other tumor cell. The R8H283 antibody contains the N-glycosylation site of human CD98hc and recognizes an epitope that is exposed upon inhibition of N-linked glycosylation. For details, see Hasegawa et al., Sci. Transl. Med. 14, eaax7706 (2022).

"N-glycosylation site of human CD98hc" refers to, among the amino acid residues constituting human CD98hc, amino acid residues to which an N-glycan binds. In an embodiment, the N-glycosylation site is an asparagine residue at position 264, 280, 323, or 405 of the amino acid sequence of SEQ ID NO: 17. In a preferable embodiment, the N-glycosylation site is an asparagine residue at position 405 of the amino acid sequence of SEQ ID NO: 17.

In an embodiment, the epitope for scFv is preferably present in the region corresponding to positions 203 to 427 of SEQ ID NO: 17 on human CD98hc. In a preferable embodiment, the epitope for scFv is preferably present in the region corresponding to positions 365 to 427 of SEQ ID NO: 17 on human CD98hc. In another preferable embodiment, the epitope for scFv is preferably present in the region corresponding to positions 365 to 409 of SEQ ID NO: 17 on human CD98hc. The phrase "corresponding to" is used here because the regions described above in human CD98hc other than isoform f are not necessarily positions 203 to 427, positions 365 to 427, and positions 365 to 409 from the amino acid residue at the N-terminus taken as position 1. The amino acid sequence at positions 365 to 427 of SEQ ID NO: 17 is as follows:
FSYGDEIGLDAAALPGQPMEAPVMLWDESSFPDIPGAVSANMTVKGQSEDPGSLLSLFRRLSDQR (SEQ ID NO: 18). The amino acid sequence at positions 365 to 409 of SEQ ID NO: 17 is as follows: FSYGDEIGLDAAALPGQPMEAPVMLW-DESSFPDIPGAVSANMTVK (SEQ ID NO: 19). Thus, in a preferable embodiment, the epitope for scFv is preferably present in the region of the amino acid sequence of SEQ ID NO: 18 of human CD98hc, and more preferably present in the region of the amino acid sequence of SEQ ID NO: 19. The amino acid sequence at positions 203 to 427 of SEQ ID NO: 17 is shown as SEQ ID NO: 20 in the sequence list.

In an embodiment, the epitope for scFv is preferably present in the region at positions 365 to 376 and in the region at positions 395 to 409 of human CD98hc. In an embodiment, the epitope for scFv preferably contains the amino acid residues at positions 374, 375, 395, 396, 397, 400, and/or 401 of human CD98hc. As used herein, the position of an amino acid residue of human CD98hc is the position on the amino acid sequence of SEQ ID NO: 17 unless indicated otherwise.

Whether an epitope for scFv is present in the region of the amino acid sequence represented by SEQ ID NO: 19 can be confirmed by the following procedure. Specifically, a polynucleotide is constructed; the polynucleotide encodes human/mouse chimera CD98hc in which the region other than the amino acid sequence represented by SEQ ID NO: 19 is replaced by amino acid sequences of mouse-originated CD98hc. The polynucleotide is then forcibly expressed in a suitable cell. Separately, a cell of the same kind in which mouse CD98hc has been forcibly expressed is prepared. The binding of the scFv to these cells is measured. When the scFv binds to the cell that expresses chimera CD98hc, but not to the cell that expresses mouse CD98hc, an epitope is determined to be present in the region. Here, the amino acid sequence of SEQ ID NO: 19 is described as an example, and measurement can also be performed in the same manner in the case of other regions or sites.

In an embodiment, the scFv preferably has a complementarity-determining region identical to at least one complementarity-determining region selected from the group consisting of three heavy-chain complementarity-determining regions (CDR-H1, CDR-H2, and CDR-H3) and three light-chain complementarity-determining regions (CDR-L1, CDR-L2, and CDR-L3) of the R8H283 antibody. The amino acid sequences of the heavy-chain and light-chain complementarity-determining regions (CDR1 to CDR3) of the R8H283 antibody are as shown in Table 1 below (Kabat).

Table 1

| Region | | SEQ ID NO | Amino Acid Sequence |
|---|---|---|---|
| Heavy Chain | CDR-H1 | SEQ ID NO: 1 | NYWMN |
| | CDR-H2 | SEQ ID NO: 2 | EIRLKSNNYATHYAESVKG |
| | CDR-H3 | SEQ ID NO: 3 | LPSFDY |
| Light Chain | CDR-L1 | SEQ ID NO: 5 | RSTKSLLHSNGNTYLY |
| | CDR-L2 | SEQ ID NO: 6 | RMSNLAS |
| | CDR-L3 | SEQ ID NO: 7 | MQHLEYPFT |

| Hinge or Spacer | Transmembrane | Signaling Domain |
|---|---|---|
| CD28 (SEQ ID NO: 37) | CD28 (SEQ ID NO: 39) | CD28 (SEQ ID NO: 41) - CD3ζ (SEQ ID NO: 44) |
| CD8α (SEQ ID NO: 38) | CD8α (SEQ ID NO: 40) | 4-1BB (SEQ ID NO: 42) - CD3ζ |
| CD8α | CD28 | CD28 - 4-1BB - CD3ζ |
| CD28 | CD28 | CD28 - CD3ζ |
| CD28 | CD8α | 4-1BB - CD3ζ |

More specifically, the scFv preferably contains:

a heavy-chain variable region containing

a heavy-chain CDR1 having the amino acid sequence of SEQ ID NO: 1;
a heavy-chain CDR2 having the amino acid sequence of SEQ ID NO: 2, and/or
a heavy-chain CDR3 having the amino acid sequence of SEQ ID NO: 3; and

a light-chain variable region containing

a light-chain CDR1 having the amino acid sequence of SEQ ID NO: 5;
a light-chain CDR2 having the amino acid sequence of SEQ ID NO: 6, and/or
a light-chain CDR3 having the amino acid sequence of SEQ ID NO: 7.

In an embodiment, CDR-H1 may have one or two amino acid substitutions, and preferably one amino acid substitution in the amino acid sequence of SEQ ID NO: 1. In an embodiment, CDR-H2 may have 1 to 3 amino acid substitutions, preferably 1 or 2 amino acid substitutions, and more preferably 1 amino acid substitution in the amino acid sequence of SEQ ID NO: 2. In an embodiment, CDR-H3 may have one or two amino acid substitutions, and preferably one amino acid substitution in the amino acid sequence of SEQ ID NO: 3. In an embodiment, CDR-L1 may have 1 to 3 amino acid substitutions, preferably 1 or 2 amino acid substitutions, and more preferably 1 amino acid substitution in the amino acid sequence of SEQ ID NO: 5. In an embodiment, CDR-L2 may have one or two amino acid substitutions, and preferably one amino acid substitution in the amino acid sequence of SEQ ID NO: 6. In an embodiment, CDR-L3 may have one or two amino acid substitutions, and preferably one amino acid substitution in the amino acid sequence of SEQ ID NO: 7. The type of amino acid substitution is not particularly limited; however, in an embodiment, the amino acid substitution is preferably a conservative amino acid substitution.

In an embodiment, the scFv contains at least one CDR selected from the group consisting of CDR-H1, CDR-H2, CDR-H3, CDR-L1, CDR-L2, and CDR-L3 of the R8H283 antibody, preferably at least 2, preferably at least 3, preferably at least 4, preferably at least 5, and preferably all of the 6 CDRs. In an embodiment, the scFv preferably contains at least CDR-H3 and/or CDR-L3 of the R8H283 antibody.

In an embodiment, the scFv preferably contains the heavy-chain CDR 1, heavy-chain CDR 2, and heavy-chain CDR 3 in this order from the N-terminus of the heavy-chain variable region. In an embodiment, the scFv preferably contains the light-chain CDR1, light-chain CDR2, and light-chain CDR3 in this order from the N-terminus of the light-chain variable region. In an embodiment, it is preferred that the scFv contains the heavy-chain CDR 1, heavy-chain CDR 2, and heavy-chain CDR 3 in this order from the N-terminus of the heavy-chain variable region, and the light-chain CDR1, light-chain CDR2, and light-chain CDR3 in this order from the N-terminus of the light-chain variable region.

In an embodiment, the heavy-chain variable region of the scFv may have the same amino acid sequence as that of the heavy-chain variable region of the R8H283 antibody (SEQ ID NO: 4). In an embodiment, the light-chain variable region of the scFv may have the same amino acid sequence as that of the light-chain variable region of the R8H283 antibody (SEQ ID NO: 8). In an embodiment, the scFv may have the same amino acid sequence as that of the heavy-chain variable region of the R8H283 antibody (SEQ ID NO: 4) and the same amino acid sequence as that of the light-chain variable region of the R8H283 antibody (SEQ ID NO: 8). As used herein, the meaning of "the same" includes the meaning of not only a complete match of amino acid sequences but also having the ability to bind to substantially the same epitope as that of the R8H283 antibody with one or several (e.g., 15 or less, preferably 10 or less, preferably 5 or less, preferably 3 or less, preferably 2) amino acid residues being different. In an embodiment, the heavy-chain variable region of the scFv may have an amino acid sequence in which the 95th isoleucine is substituted with arginine (I95R) in the amino acid sequence of SEQ ID NO: 4. In an embodiment, the light-chain variable region of the scFv may have an amino acid sequence in which the 12th proline is substituted with arginine (P12R) in the amino acid sequence of SEQ ID NO: 8. In an embodiment, the light-chain variable region of the scFv may have an amino acid sequence in which the 90th isoleucine is

substituted with arginine (I90R) in the amino acid sequence of SEQ ID NO: 8. In an embodiment, the light-chain variable region of the scFv may have an amino acid sequence in which the 12th proline is substituted with glutamine (P12Q) in the amino acid sequence of SEQ ID NO: 8. These amino acid substitutions can be combined in any manner. For example, the scFv may have I95R substitution in the heavy-chain variable region and P12R and I90R substitutions in the light-chain variable region, or may have I95R substitution in the heavy-chain variable region and P12Q and I90R substitutions in the light-chain variable region.

In an embodiment, the amino acid sequence of the heavy-chain variable region of the scFv has at least 90%, preferably at least 95%, preferably at least 98%, and preferably at least 99% identity to the amino acid sequence of SEQ ID NO: 4. In an embodiment, the light-chain variable region of the scFv has at least 90%, preferably at least 95%, preferably at least 98%, and preferably at least 99% identity to the amino acid sequence of SEQ ID NO: 8. The identity of amino acids sequences is measured by the technique described below.

When the scFv contains at least one amino acid sequence selected from the group consisting of SEQ ID NOs: 1 to 8, the at least one amino acid sequence may contain any mutation as long as the epitope for the antibody is present within the region affected by the presence of an N-glycan on human CD98hc. In an embodiment, there is preferably no mutation in the complementarity-determining region of the scFv. This is because a mutation in the complementarity-determining region is generally considered to affect the epitope. Thus, in an embodiment, a mutation in the variable region is preferably present in a heavy-chain FR region and/or a light-chain FR region.

In an embodiment, the amino acid sequence of a heavy-chain FR region of the heavy-chain variable region of the scFv has at least 80%, preferably at least 90%, preferably at least 95%, preferably at least 98%, and preferably at least 99% identity to the amino acid sequence of a heavy-chain FR region of the amino acid sequence of SEQ ID NO: 4. For example, the amino acid sequence of the heavy-chain FR1 region of the heavy-chain variable region of the scFv has at least 80%, preferably at least 90%, preferably at least 95%, preferably at least 98%, and preferably at least 99% identity to the amino acid sequence of the heavy-chain FR1 region of the amino acid sequence of SEQ ID NO: 4. For example, the amino acid sequence of the heavy-chain FR2 region of the heavy-chain variable region of the scFv has at least 80%, preferably at least 90%, preferably at least 95%, preferably at least 98%, and preferably at least 99% identity to the amino acid sequence of the heavy-chain FR2 region of the amino acid sequence of SEQ ID NO: 4. For example, the amino acid sequence of the heavy-chain FR3 region of the heavy-chain variable region of the scFv has at least 80%, preferably at least 90%, preferably at least 95%, preferably at least 98%, and preferably at least 99% identity to the amino acid sequence of the heavy-chain FR3 region of the amino acid sequence of SEQ ID NO: 4. For example, the amino acid sequence of the heavy-chain FR4 region of the heavy-chain variable region of the scFv has at least 80%, preferably at least 90%, preferably at least 95%, preferably at least 98%, and preferably at least 99% identity to the amino acid sequence of the heavy-chain FR4 region of the amino acid sequence of SEQ ID NO: 4. In an embodiment, the amino acid sequence of a light-chain FR region of the light-chain variable region of the scFv has at least 80%, preferably at least 90%, preferably at least 95%, preferably at least 98%, and preferably at least 99% identity to the amino acid sequence of a light-chain FR region of the amino acid sequence of SEQ ID NO: 8. For example, the amino acid sequence of the light-chain FR1 region of the light-chain variable region of the scFv has at least 80%, preferably at least 90%, preferably at least 95%, preferably at least 98%, and preferably at least 99% identity to the amino acid sequence of the light-chain FR1 region of the amino acid sequence of SEQ ID NO: 8. For example, the amino acid sequence of the light-chain FR2 region of the light-chain variable region of the scFv has at least 80%, preferably at least 90%, preferably at least 95%, preferably at least 98%, and preferably at least 99% identity to the amino acid sequence of the light-chain FR2 region of the amino acid sequence of SEQ ID NO: 8. For example, the amino acid sequence of the light-chain FR3 region of the light-chain variable region of the scFv has at least 80%, preferably at least 90%, preferably at least 95%, preferably at least 98%, and preferably at least 99% identity to the amino acid sequence of the light-chain FR3 region of the amino acid sequence of SEQ ID NO: 8. For example, the amino acid sequence of the light-chain FR4 region of the light-chain variable region of the scFv has at least 80%, preferably at least 90%, preferably at least 95%, preferably at least 98%, and preferably at least 99% identity to the amino acid sequence of the light-chain FR4 region of the amino acid sequence of SEQ ID NO: 8.

The number of mutations introduced into the at least one amino acid sequence selected from the group consisting of SEQ ID NOs: 1 to 8 (amino acid residue conversion) is not particularly limited. In an embodiment, the identity of the amino acid sequence before mutation introduction with the amino acid sequence after mutation introduction is at least 70%, preferably at least 75%, preferably at least 80%, preferably at least 85%, preferably at least 90%, preferably at least 95%, preferably at least 96%, preferably at least 97%, preferably at least 98%, and preferably at least 99%.

The identity of amino acids can be calculated with a commercially available analytical tool or an analytical tool available through the Internet (e.g., software such as FASTA, BLAST, PSI-BLAST, or SSEARCH). For example, the main initial conditions generally applied for a BLAST search are as described below. Specifically, a value (%) of the identity of an amino acid sequence with another sequence can be calculated by performing a search on Advanced BLAST 2.1 with blastp for the program, with the expect value set to 10, all filters turned off, BLOSUM62 used for matrix, the gap existence cost, per residue gap cost, and lambda ratio set to 11, 1, and 0.85 (default values), respectively, and other various parameters also set to default values. The identity percentage is determined by rounding off.

The mutation introduced into an amino acid sequence described above is, for example, substitution, deletion, or insertion. Specific introduction of mutations is not particularly limited as long as it can be achieved using a conventional method. The substitution of an amino acid is preferably a conservative substitution. "Conservative substitution" refers to replacement of an amino acid residue by another amino acid residue having a side chain similar to the side chain of the replaced amino acid residue.

Specific conservative amino acid substitutions include the following: substitution between amino acid residues each having a basic side chain, such as lysine, arginine, and histidine; substitution between amino acid residues each having an acidic side chain, such as aspartic acid and glutamic acid; substitution between amino acid residues each having an uncharged polar side chain, such as glycine, asparagine, glutamine, serine, threonine, tyrosine, and cysteine; substitution between amino acid residues each having a nonpolar side chain, such as alanine, valine, leucine, isoleucine, proline, phenylalanine, methionine, and tryptophan; substitution between amino acid residues each having a β-branched side chain, such as threonine, valine, and isoleucine; and substitution between amino acid residues each having an aromatic side chain, such as tyrosine, phenylalanine, tryptophan, and histidine.

In an embodiment, the scFv is preferably a humanized scFv. Humanized scFv can be obtained, for example, by replacing the amino acid sequence of a region other than the complementarity-determining region of a mouse antibody with the amino acid sequence of a human antibody. The region other than the complementarity-determining region present in the variable regions is referred to as "FR." The heavy-chain variable region and the light-chain variable region each have FR1 region between the N-terminus of the variable region and CDR1, FR2 region between CDR1 and CDR2, FR3 region between CDR2 and CDR3, and FR4 region between CDR3 and the C-terminus. At least one or all of these regions can be humanized to obtain a humanized scFv. The amino acid sequence of human origin usually accounts for about 90% or more of a humanized scFv, but this is not limited.

In an embodiment, the scFv is preferably a human scFv. A human scFv is an scFv the entire structure of which is derived from humans. A human scFv is sometimes referred to as a "fully humanized scFv."

The length of the hinge or spacer sequence and the type of amino acid residues constituting the hinge or spacer sequence are not limited as long as they do not inhibit the functionality of the chimeric antigen receptor. For example, the hinge or spacer sequence can be designed so as to be composed of about 10 to 250, 10 to 200, 10 to 100, 10 to 50, or 10 to 25 amino acid residues. For example, the hinge or spacer sequence may be, but not particularly limited to, the sequence of a part of a factor used in a transmembrane domain (e.g., CD28, CD8α, IgG1, IgG4, IgD, CD34, and 4-1BB) or a constant region containing the hinge region of an antibody (e.g., IgG4 hinge-CH2-CH3), as described below.

The type of the transmembrane domain is not limited as long as it does not inhibit the functionality of the chimeric antigen receptor. For example, CD28, CD3ε, CD8α, CD3, CD4, or 4-1BB can be used. These transmembrane domains may be appropriately mutated as long as the mutation does not inhibit the functionality of the chimeric antigen receptor. In an embodiment, the transmembrane domain is preferably CD28 or CD8α.

The intracellular signaling domain can be derived from, for example, CD3, also known as a "TCR ζ chain." CD3 may be mutated as long as the mutations do not inhibit the functionality of the chimeric antigen receptor. Introduction of mutation into CD3 is preferably performed so as to incorporate an immunoreceptor tyrosine-based activation motif (ITAM). In an embodiment, the intracellular signaling domain is preferably CD28-CD3ζ, 4-1BB-CD3ζ, or CD28-4-1BB-CD3ζ.

The combination of the hinge or spacer sequence, the transmembrane domain, the intracellular signaling domain, etc. can be any combination. In an embodiment, it is preferred to use any of the following combinations:

In an embodiment, the chimeric antigen receptor may contain other regions in addition to those described above. Examples of other regions include a costimulatory factor domain, a leader sequence for promoting transport of the chimeric antigen receptor onto the cell membrane (signal peptide), and a spacer or linker sequence (e.g., between the transmembrane domain and the intracellular signaling domain).

The costimulatory factor is not particularly limited as long as it is a costimulatory factor possessed by cells such as T cells. For example, one or more costimulatory factors selected from the group consisting of OX40, 4-1BB, CD28, etc. can be appropriately selected and used. These costimulatory factors may be mutated as long as the mutations do not inhibit the functionality of the chimeric antigen receptor.

Techniques for producing chimeric antigen receptors are known. For example, the methods described in NPL 1 to 4 can be mentioned. The chimeric antigen receptor can be produced in accordance with or in line with a known method.

## 2. Polynucleotide

In an embodiment, a polynucleotide encoding the chimeric antigen receptor described above is provided. The term "polynucleotide" refers to a high-molecular compound composed of linearly polymerized nucleotides (e.g., ribonucleotides or deoxyribonucleotides). The polynucleotide may be a single-stranded or double-stranded chain.

The base sequence of the polynucleotide may be any sequence that can encode the chimeric antigen receptor, and is not particularly limited.

The base sequences of polynucleotides encoding the amino acid sequences of the heavy-chain and light-chain

complementarity-determining regions and variable regions of the R8H283 antibody are indicated as SEQ ID NOs: 9 to 16. Table 2 below shows the correspondence of these sequences.

Table 2

| Region | | Amino Acid Sequence | Base Sequence |
|---|---|---|---|
| Heavy Chain | CDR-H1 | SEQ ID NO: 1 | SEQ ID NO: 9 |
| | CDR-H2 | SEQ ID NO: 2 | SEQ ID NO: 10 |
| | CDR-H3 | SEQ ID NO: 3 | SEQ ID NO: 11 |
| | Variable Region | SEQ ID NO: 4 | SEQ ID NO: 12 |
| Light Chain | CDR-L1 | SEQ ID NO: 5 | SEQ ID NO: 13 |
| | CDR-L2 | SEQ ID NO: 6 | SEQ ID NO: 14 |
| | CDR-L3 | SEQ ID NO: 7 | SEQ ID NO: 15 |
| | Variable Region | SEQ ID NO: 8 | SEQ ID NO: 16 |

The polynucleotide encoding the scFv contains at least one base sequence selected from the group consisting of SEQ ID NOs: 9 to 16 (the combination of two or more sequences can be of any combination). In a preferable embodiment, the polynucleotide encoding the scFv contains at least one base sequence selected from the group consisting of SEQ ID NOs: 9 to 11 (preferably 2 or more, and more preferably all) and/or at least one base sequence selected from the group consisting of SEQ ID NOs: 13 to 15 (preferably 2 or more, and more preferably all). In a preferable embodiment, the polynucleotide encoding the scFv contains the base sequences of SEQ ID NOs: 9 to 11 and the base sequences of SEQ ID NOs: 13 to 15. In a preferable embodiment, the polynucleotide encoding the scFv contains the base sequence of SEQ ID NO: 12 and/or the base sequence of SEQ ID NO: 16.

The base sequence of the polynucleotide encoding the scFv does not have to be a complete match with SEQ ID NOs: 9 to 16 as long as the polynucleotide encodes the scFv. For example, the base sequence of the polynucleotide encoding the scFv may have at least 80%, preferably, at least 85%, preferably at least 90%, preferably at least 95%, preferably at least 98%, and preferably at least 99% identity to SEQ ID NOs: 9 to 16. The identity can be calculated using a commercially available analytical tool or an analytical tool available through telecommunication lines (the Internet). Specifically, the value (%) of the homology of nucleotide sequences can be calculated, for example, by performing a search using blastn for the program in Advanced BLAST 2.1, with various parameters set to default values. The frequency of codon usage for the polynucleotide may be optimized according to the type of the vector or cell expressing it.

The state of the polynucleotide is not particularly limited; for example, the polynucleotide may be an isolated polynucleotide or a polynucleotide incorporated in a vector. The type and use of the vector are not particularly limited. For example, the vector can be a plasmid vector or a virus vector (e.g., an adenovirus, retrovirus, or lentivirus). The vector can be, for example, a vector for cloning or for expression. The vector for expression includes vectors for prokaryotic cells, such as *Escherichia coli*, and actinomycete, and vectors for eukaryotic cells, such as yeast cells, insect cells, and mammalian cells.

3. Cells

In an embodiment, a cell containing a polynucleotide encoding the chimeric antigen receptor is provided. The cell can be of any type; however, a cell having cytotoxic activity is preferable. Examples of cells having cytotoxic activity include T cells, NK cells, and NK-T cells, with killer T cells (which are also referred to as "cytotoxic T cells" or "CTL") being preferable.

The cell may contain the polynucleotide in the form of a vector, or may contain the polynucleotide integrated into genomic DNA within the cell. The cell may be in the state of expressing the polynucleotide encoding the chimeric antigen receptor, or in the state of not expressing the polynucleotide. When the polynucleotide encoding the chimeric antigen receptor is being expressed in the cell, the scFv region constituting the chimeric antigen receptor is preferably exposed on the outside of the cell, and the transmembrane domain and the intracellular signaling domain are preferably present in the cell membrane or inside the cell.

When the scFv region recognizes its epitope, the scFv region intracellularly activates a signal for evoking cytotoxic activity through the transmembrane domain. In conjunction with this, the cell attacks against other cells or tissues expressing the epitope or exerts its cytotoxic activity on the cells or tissues.

A cell containing the polynucleotide encoding the chimeric antigen receptor (in particular, cells having cytotoxic activity) is useful as an active ingredient of pharmaceutical compositions.

A cell containing the polynucleotide encoding the chimeric antigen receptor (e.g., a CAR-T cell) can be produced with reference to, for example, the method disclosed in Non-patent Literature 1 to 4.

For example, a polynucleotide encoding the chimeric antigen receptor may be introduced into a cell by using a

vector such as a retroviral vector or a lentiviral vector.

Such cells containing a polynucleotide encoding the chimeric antigen receptor recognize an epitope that contains an N-glycosylation site of human CD98hc and that is exposed by inhibiting N-linked glycosylation to thereby specifically recognize myeloma cells, myeloma progenitor cells, and other tumor cells; thus, these cells are useful for the treatment and/or prevention of tumors. The tumor can be of any type, and includes solid cancer and blood cancer. Examples of solid cancer include lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, and rhabdomyosarcoma. Examples of blood cancer include leukemia, diseases involving neoplastic growth of plasma cells (e.g., multiple myeloma), and malignant lymphoma. In an embodiment, leukemia is preferably lymphatic leukemia.

4. Pharmaceutical Composition and Method for Treatment and/or Prevention

In an embodiment, a pharmaceutical composition containing a cell containing a polynucleotide encoding the chimeric antigen receptor, and a method for treating and/or preventing a disease using a cell containing a polynucleotide encoding the chimeric antigen receptor are provided.

The content of the cell in the pharmaceutical composition can be suitably determined taking into consideration the type of the target disease, intended treatment effect, administration method, treatment period, patient's age, patient's body weight, etc. The content of the cell in the pharmaceutical composition can be, for example, about $10^1$ to $10^7$ cells/mL.

The administration form of the pharmaceutical composition is not particularly limited as long as a desired effect is achieved. The composition can be administered to mammals, including humans, through an administration route, either peroral administration or parenteral administration, (e.g., intravenous injection, intramuscular injection, subcutaneous administration, rectal administration, transdermal administration, and local administration). Because the active ingredient is a cell, the preferable administration form is parenteral administration, and more preferably intravenous injection. The dosage form and production method of the composition for peroral administration or parenteral administration would be well known to a person skilled in the art, and the pharmaceutical composition in any dosage form can be produced in accordance with a conventional method by mixing cells containing a polynucleotide encoding the chimeric antigen receptor with a pharmaceutically acceptable carrier and the like.

The dosage form of the composition for parenteral administration includes injectable drugs (e.g., drip-injectable drugs, intravenously injectable drugs, intramuscularly injectable drugs, subcutaneously injectable drugs, and intradermally injectable drugs), drugs for external use (e.g., ointments, cataplasms, and lotions), suppositories, inhalants, eye drops, eye ointments, nasal drops, ear drops, and liposome drugs. For example, an injectable drug can be prepared by dissolving the cells in injectable distilled water; and a solubilizing agent, a buffer, a pH adjuster, a tonicity agent, a soothing agent, a preservative, a stabilizer, and the like can be optionally added thereto. The pharmaceutical composition may be in the form of freeze-dried formulation, which will be prepared when used.

The pharmaceutical composition may further contain other medicinal agents effective in treatment or prevention of diseases. The pharmaceutical composition may also optionally contain components such as a sterilizer, an anti-phlogistic, a cellular stimulant, vitamins, and an amino acid.

For the carrier used in preparing a drug of the pharmaceutical composition, an excipient, a binder, a disintegrant, a lubricant, a colorant, and a flavoring agent typically used in the art can be used; and a stabilizer, an emulsifier, an absorption promoter, a surfactant, a pH adjuster, an antiseptic, an antioxidant, a filler, a moisture agent, a surface activation agent, a dispersant, a buffer, a preservative, a solubilizing agent, a soothing agent, and the like can also optionally be used.

The disease treated or prevented using the pharmaceutical composition can be of any type as long as the treatment or prevention can be achieved. Examples of specific target diseases include tumors. The tumor can be of any type, and includes solid cancer and blood cancer. Examples of solid cancer include lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, and rhabdomyosarcoma. Examples of blood cancer include leukemia, diseases involving neoplastic growth of plasma cells (e.g., multiple myeloma), and malignant lymphoma. In an embodiment, leukemia is preferably lymphatic leukemia. In an embodiment, a preferable disease is a disease that causes neoplastic growth of plasma cells. A "disease that causes neoplastic growth of plasma cells" is a disease characterized by abnormal neoplastic growth of plasma cells and an increase in abnormal protein secreted by the plasma cells. Examples of these diseases include multiple myeloma, plasma cell leukemia, plasmacytoma, H-chain disease, and systemic AL amyloidosis. In an embodiment, a preferable target disease is multiple myeloma.

The administration target (test subject) of the pharmaceutical composition is, for example, an animal having any of the diseases described above or an animal with a potential to develop such a disease. A "potential to develop such a disease" can be determined, for example, by the diagnostic method described below. The animal is, for example, a mammal, and preferably a human.

The dose of the pharmaceutical composition can be determined by a clinical physician, taking into consideration

various factors, such as administration route, the type of disease, the degree of symptoms, patient's age, gender, body weight, severity of the disease, pharmacological findings such as pharmacokinetics and toxicological characteristics, use or non-use of a drug delivery system, and whether the composition is administered as part of a combinational drug with other medicinal agents. The dosage of the pharmaceutical composition can be, for example, about $10^4$ to $10^9$ cells/kg (body weight). The administration schedule of the pharmaceutical composition can also be determined taking into consideration the same factors as those for the dose. For example, the composition can be administered once every day to once every month in the daily dose described above.

As described above, the cell containing a polynucleotide encoding the chimeric antigen receptor can be used in producing the pharmaceutical composition described above.

In the present specification, the term "comprising" includes "consisting essentially of" and "consisting of." The present disclosure also encompasses any and all combinations of the technical elements described in the specification.

Furthermore, the various characteristics (properties, structures, functions, etc.) described in each embodiment of the present disclosure above may be combined in any manner to specify the subject matter encompassed by the disclosure.

In other words, the present disclosure encompasses all subject matter formed of any and all combinations of the combinable characteristics described in the present specification.

Examples

The subject matter encompassed by the present disclosure is described below in more detail. However, the subject matter is not limited to the following Examples. In the following description, unless otherwise specified, the experiments were performed at room temperature under atmospheric pressure. Unless otherwise specified, "%" means volume percent concentration (v/v %).

Example 1: Effect of Order of VL and VH on CD98 CAR-T Cell Activity

The inventors previously identified R8H283 antibody, which specifically binds to myeloma cells in the bone marrow of human multiple myeloma patients (PTL 1). R8H283 is an antibody that recognizes human CD98hc, and the amino acid sequences of the heavy-chain and light-chain complementarity-determining regions (CDR1 to CDR3) are as shown below (the complementarity-determining regions were determined by using the Kabat).

Table 3

| Region | | SEQ ID NO | Amino Acid Sequence |
|---|---|---|---|
| Heavy Chain | CDR-H1 | SEQ ID NO: 1 | NYWMN |
| | CDR-H2 | SEQ ID NO: 2 | EIRLKSNNYATHYAESVKG |
| | CDR-H3 | SEQ ID NO: 3 | LPSFDY |
| Light Chain | CDR-L1 | SEQ ID NO: 5 | RSTKSLLHSNGNTYLY |
| | CDR-L2 | SEQ ID NO: 6 | RMSNLAS |
| | CDR-L3 | SEQ ID NO: 7 | MQHLEYPFT |

The use of the scFv derived from R8H283 enabled CD98 CAR-T cells to capture target cells. To exert a drug efficacy on target cells, proper capture is necessary.

During the production of scFvs, the effect of the order of VH (heavy-chain variable region) and VL (light-chain variable region) on the drug efficacy of CD98 CAR-T cells was evaluated. CAR-T cell therapies currently on the market include CD19 CAR-T cells and BCMA CAR-T cells. Of these, the scFvs of scFv-incorporated CAR-T cells all contained VL-VH in this order (e.g., KYMRIAH, YESCARTA, Breyanzi, and Abecma (all product names)).

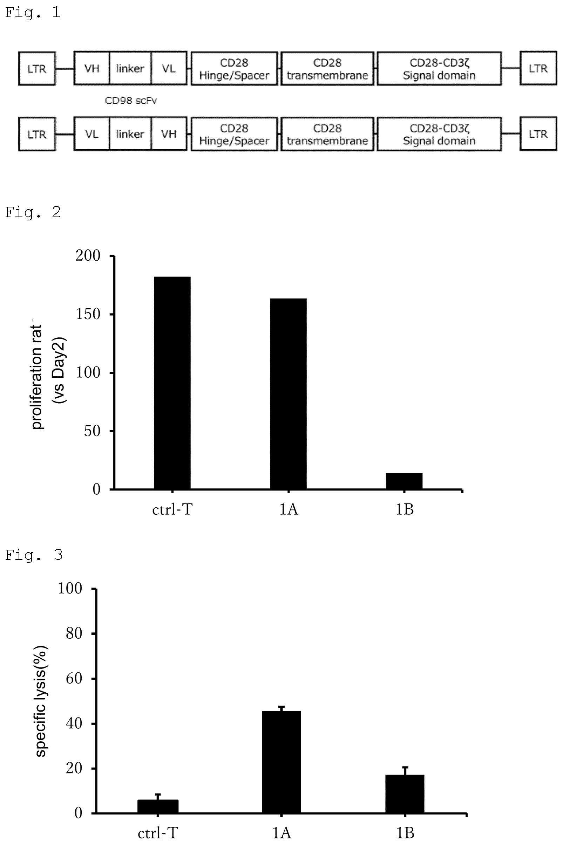

Table 4 below and Fig. 1 show CAR-T cells for evaluating the order of VH and VL, prepared by using a retroviral vector.

Table 4

| | scFv Order | Linker Connecting VH to VL | Hinge or Spacer | Transmembrane | Signaling Domain |
|---|---|---|---|---|---|
| CAR-T 1A<br>DNA: SEQ ID NO: 45<br>AA: SEQ ID NO: 104 | VH-VL | 218 Linker | CD28 | CD28 | CD28 - CD3ζ |
| CAR-T 1B<br>DNA: SEQ ID NO: 46<br>AA: SEQ ID NO: 105 | VL-VH | 218 Linker | CD28 | CD28 | CD28 - CD3ζ |

For the production of retroviral vectors and the production of CAR-T cells using retroviral vectors, the method of Hosen et al. (NPL 4) was referenced. Specifically, 293T (ATCC: CRL-3216) was cultured in a 10 cm dish at 37°C in 5% $CO_2$ using D-MEM supplemented with 10% fetal bovine serum (FBS) (Nichirei, Lot 19C00A or Lot S173012) and 1% penicillin-streptomycin. After the cells were detached, the cell count was measured by trypan blue staining, and the cells were then seeded at $5 \times 10^6$ cells/dish. For retroviral vector production, after one day from cell seeding, a Gag-pol vector, a VSV-G vector, a CAR vector, Opti-MEM, and Lipofectamin 2000 (Thermo Fisher Scientific, 11668019) were mixed and added dropwise to the cells for transfection. After 48 hours from transfection, viruses were collected and cryopreserved at -80°C. Subsequently, cryopreserved, commercially available human PBMCs (Precision for Medicine, Inc., 39000-10M, Lot#3010116166) were rapidly thawed in a water bath at 37°C, suspended in GT-T551 (Takara, WK551S) supplemented with 5% human albumin serum and a 1% penicillin-streptomycin mixture solution, and centrifuged to remove the supernatant (400 × g, 5 min, 25°C). The commercially available human PMBCs were stimulated with RetroNectin (Takara, T100B) in a 48-well plate in which anti-CD3 antibody (Clone; OKT3) and anti-CD28 antibody were immobilized. After one day from PBMC stimulation, T cells were expanded in medium supplemented with IL-2 (100 U/mL). After two days from PBMC stimulation, a CAR sequence was introduced by using a retroviral vector using RetroNectin with reference to the protocol supplied with the RetroNectin. Thereafter, the cells were reseeded in fresh medium every two to three days so as to reach a cell density of $5 \times 10^5$ cells/mL for expansion. After nine days from PBMC stimulation, the cells were harvested and cryopreserved at -80°C.

CAR-T 1B, which contained an scFv produced in the VL-VH order, showed limited CAR-T cell proliferation compared with CAR-T 1A, which contained an scFv produced in the VH-VL order (Fig. 2). Although the inventors attempted to improve performance by using CAR constructs prepared in the VL-VH order with two different leader sequences, the proliferation was similar to that of CAR-T 1B. One of the leader sequences contained the amino acid sequence of SEQ ID NO: 23. Note that the amino acid sequence of SEQ ID NO: 23 does not contain the initiating methionine residue.

Example 2: In Vitro Activity Evaluation

The activity of the CAR-T cells prepared in Example 1 was evaluated as follows.

The cytotoxic activity was measured according to the following procedure. NALM6-Luc cells ($1 \times 10^5$ cells) (NALM6, clone G5 (ATCC, CRL-3273) transfected to stably express a luciferase) and CAR-T cells were incubated in a 96-well plate for 4 hours. Thereafter, VivoGlo-Luciferin (Promega) was added to the cell culture medium at a final concentration of 140 μg/mL, and the suspension was transferred to a 1/2 Area OptiPlate-96 (Revvity). Luminescence was measured with an Infinite M1000 PRO (Tecan). The cytotoxic activity was then calculated according to the following formula.

$$\text{Specific Lysis (\%)} = [1 - \text{Luminescence (CAR-T:NALM6-Luc = 1)} / \text{Luminescence (CAR-T:NALM6-Luc = 0)}] \times 100$$

The interferon (IFN)-γ production ability was evaluated according to the following procedure. NALM6-Luc cells and CAR-T cells were mixed in an amount of $1 \times 10^5$ cells for each type in a 96-well plate. After 4 hours, the plate was centrifuged at 1500 rpm for 3 minutes at 4°C to collect 100 μL of the supernatant. The amount of cytokines in the collected supernatant was measured according to the protocol for the Human IFN-gamma Quantikine ELISA Kit (R&D SYSTEMS).

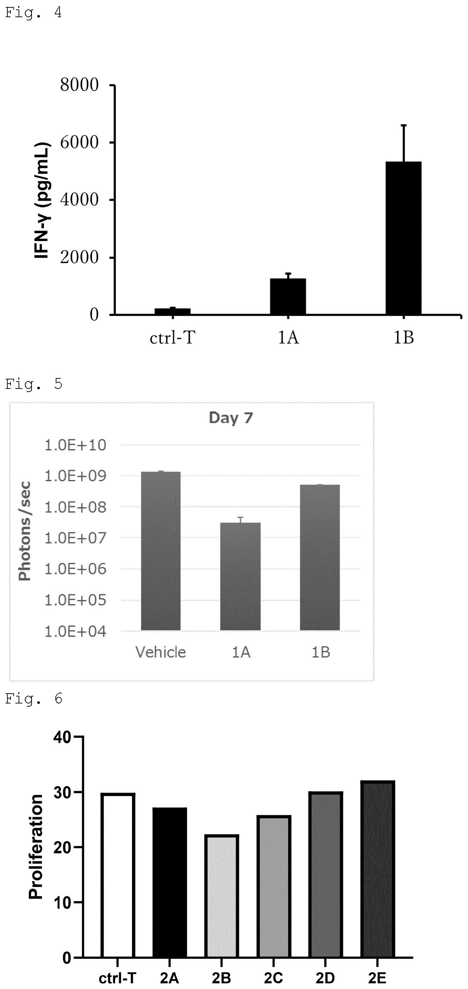

The results indicate that CAR-T 1A and CAR-T 1B had equivalent cytotoxic activity (Fig. 3), while CAR-T 1B had a higher IFN-γ production ability than CAR-T 1A (Fig. 4).

Example 3: In Vivo Evaluation

Because a certain degree of activity was shown in vitro despite a low proliferation ability, CAR-T 1B was evaluated in vivo using a NALM6-Luc xenograft model according to the following procedure. NOD/Shi-scid, IL-2RγKO Jic (NOG) mice (In-Vivo Science Inc.) at 6 weeks of age, produced by CLEA Japan Inc., were purchased. The day before transplantation, 20 mg/kg Busulfex (Otsuka Pharmaceutical Co., Ltd.) was administered intraperitoneally to the mice at 7 weeks of age. The next day, NALM6-Luc cells at $1 \times 10^5$ cells/0.1 mL/body as tumor cells were transplanted into the tail vein to prepare tumor-bearing mice. After three days from transplantation, the luminescence was measured with an IVIS Lumina X5 (Revvity), and the luminescence value was used as an index for group division based on stratified random sampling using SAS software R9.4 (SAS Institute Japan Ltd.). The CAR-T cells were administered at 0.1 mL/body via the tail vein according to the group composition once on the day the mice were divided into groups. Drug efficacy was evaluated using the tumor luminescence value measured with the IVIS Lumina X5 as an index. For tumor luminescence values, 15 mg/mL VivoGlo-luciferin was intraperitoneally administered at a dose of 10 mL/kg based on the body weight measured beforehand, and the tumor luminescence value was measured after 20 minutes from administration. Total flux [p/s] of a tumor luminescence value means photons/sec.

CAR-T cells were prepared to be $1 \times 10^6$ CAR-T cells and intravenously injected into the tail at a dose of 0.1 mL/body once on the same day of grouping. The results indicate that CAR-T 1B, which contained an scFv produced in the VL-VH order, showed limited activity. On the other hand, CAR-T 1A, which contained an scFv produced in the VH-VL order, showed clear antitumor activity (Fig. 5). This indicates that to allow CD98 CAR-T cells to exert their drug efficacy, it is not sufficient to simply prepare an scFv of VL-VH order, by referring to conventional CAR-T cells.

Example 4: Effect of Linker Length on CD98 CAR-T Cell Activity 1

The effect of the length of the linker connecting VL and VH of an scFv on the drug efficacy of CD98 CAR-T cells was evaluated. Conventionally known linkers for use include a 218 linker (18 amino acids) and a G4S linker (15 amino acids). However, because the results above suggested that CD98 CAR-T cells would not bring about effects with conventional methods, the drug efficacy of CD98 CAR-T cells was evaluated by replacing the linker portion of the CD98 CAR-T cells with a 218 linker or with a G4S linker of a different length (10, 15, 20, or 25 amino acids). Table 5 shows CAR-T cells produced with a lentiviral vector for evaluating the effect of the linker length on CD98 CAR-T cells.

Table 5

| | scFv Order | Linker Connecting VH to VL | Hinge or Spacer | Transmembrane | Signaling Domain |
|---|---|---|---|---|---|
| CAR-T 2A<br>DNA: SEQ ID NO: 47<br>AA: SEQ ID NO: 106 | VH-VL | 218 Linker SEQ ID NO: 28 | CD28 | CD28 | CD28 - CD3ζ |
| CAR-T 2B<br>DNA: SEQ ID NO: 48<br>AA: SEQ ID NO: 107 | VH-VL | G4S×2 (10 Amino Acids) SEQ ID NO: 29 | CD28 | CD28 | CD28 - CD3ζ |
| CAR-T 2C<br>DNA: SEQ ID NO: 49<br>AA: SEQ ID NO: 108 | VH-VL | G4S×3 (15 Amino Acids) SEQ ID NO: 30 | CD28 | CD28 | CD28 - CD3ζ |
| CAR-T 2D<br>DNA: SEQ ID NO: 50<br>AA: SEQ ID NO: 109 | VH-VL | G4S×4 (20 Amino Acids) SEQ ID NO: 31 | CD28 | CD28 | CD28 - CD3ζ |
| CAR-T 2E<br>DNA: SEQ ID NO: 51<br>AA: SEQ ID NO: 110 | VH-VL | G4S×5 (25 Amino Acids) SEQ ID NO: 32 | CD28 | CD28 | CD28 - CD3ζ |

Lentiviral vectors were produced according to the instructions included with the Lentiviral High Titer Packaging Mix (Takara Code: 6194, Takara Bio Inc.) or LVpro Packaging Mix with pLVpro (Takara Code: 6195, Takara Bio Inc.).

Specifically, 293T (ATCC) was cultured in a 10 cm dish at 37°C in 5% $CO_2$ using D-MEM supplemented with 10% FBS and 1% penicillin-streptomycin. After the cells were detached, the cell count was measured by trypan blue staining, and the cells were then seeded at $5 \times 10^6$ cells/dish. For lentiviral vector production, after one day from cell seeding,

Lentiviral High Titer Packaging Mix or LVpro Packaging Mix with pLVpro, a CAR vector, serum-free D-MEM, and TransIT-293 Transfection Reagent (Takara, V2700) were mixed and added dropwise to the cells for transfection. When the LVpro Packaging Mix with pLVpro was used, the medium was replaced with 10% FBS and 1% penicillin-streptomycin D-MEM after 24 hours from transfection. After 48 hours from transfection, viruses were collected, concentrated, and cryopreserved at -80°C. Subsequently, for the protocol for producing CAR-T cells using a lentiviral vector, Engineering of CAR T cells for research use provided by Miltenyi Biotec was referenced.

Specifically, cryopreserved, commercially available human PBMCs were rapidly thawed in a water bath at 37°C, suspended in TexMACS (Miltenyi Biotec) supplemented with 5% human albumin serum and a 1% penicillin-streptomycin mixture solution, and centrifuged to remove the supernatant (400 $\times$ g, 5 min, 25°C). The commercially available human PMBCs were stimulated with TransAct (Miltenyi Biotec) and then further expanded in medium supplemented with 12.5 ng/mL IL-7 (Miltenyi Biotec) and 12.5 ng/mL IL-15 (Miltenyi Biotec) to induce T cells. On day 2 from PBMC stimulation, a sequence carrying the CD98 CAR structure was introduced by using a lentiviral vector (Fig. 1). Thereafter, the cells were reseeded in fresh medium every two to three days so as to reach a cell density of $5 \times 10^5$ cells/mL. During this process, dasatinib (Tokyo Chemical Industry Co. Ltd.) was added to a final concentration of 1 $\mu$M from day 6 after PBMC stimulation. On day 9 from PBMC stimulation, the cells were harvested and cryopreserved at -80°C.

A continuous antigen stimulation test was performed as follows. CAR-positive T cells ($0.8 \times 10^6$ cells) and NALM6-Luc cells ($1.6 \times 10^6$ cells) were suspended in a mixed medium (a medium of a mixture of TexMACS supplemented with 5% human albumin serum and a 1% penicillin-streptomycin mixed solution and RPMI1640 supplemented with 10% FBS (Nichirei, Lot 19C00A) and 1% penicillin-streptomycin; TexMACS:RPMI1640 = 1:1) and co-cultured in a T25 flask. After 72 to 96 hours, the cells were harvested and prepared to be the cell count of CAR-positive cells mentioned above. Then, co-culture was started again with NALM6-Luc cells ($1.6 \times 10^6$ cells) that had been cultured separately. Some of the harvested cells mentioned above were also subjected to a cytotoxic activity test. These tests were continued until the point at which CAR-T cell proliferation was no longer observed.

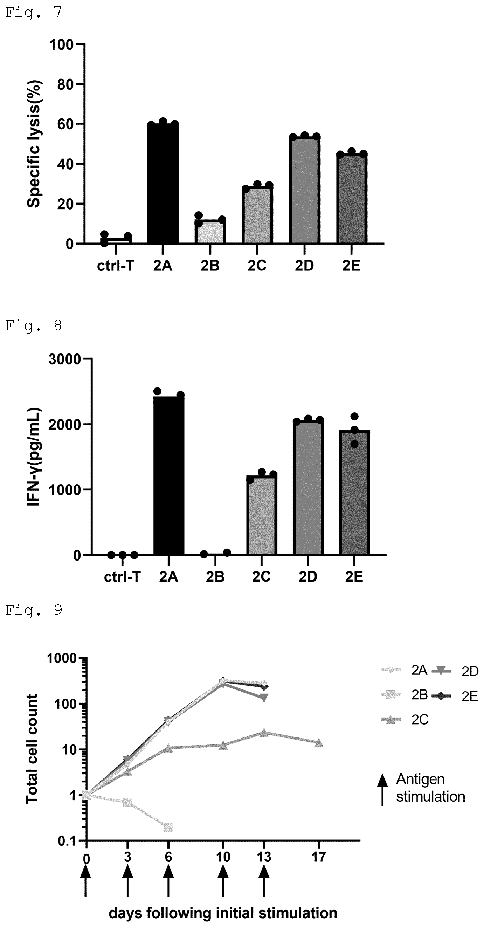

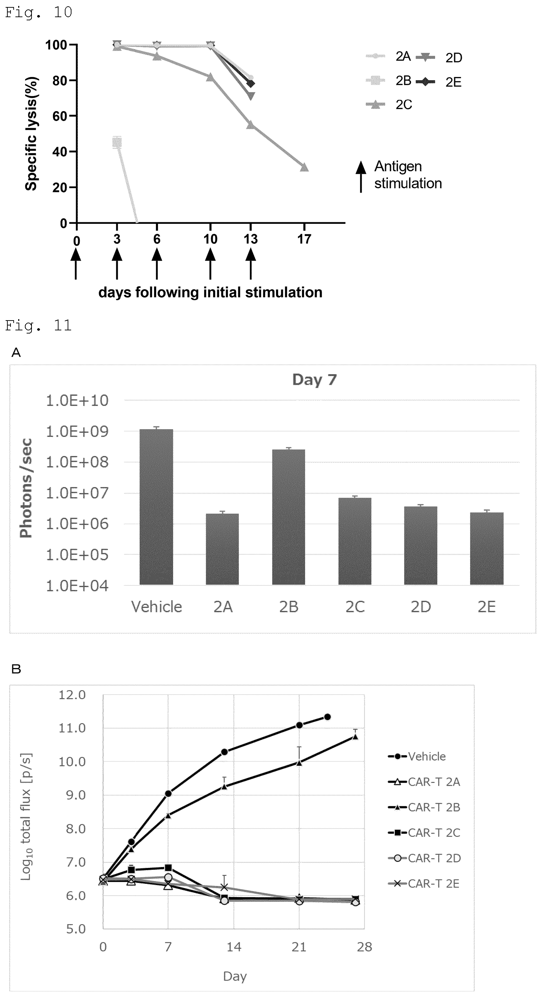

The results of this study indicated that the linker length made no significant differences in cell proliferation during the production of CAR-T cells (Fig. 6). Compared with CAR-T 2A with the 218 linker, CAR-T 2B and CAR-T 2C, respectively with a linker length of 10 and 15 amino acids, had lower cytotoxic activity and lower IFN-$\gamma$ production ability (Figs. 7 and 8). Similar trends were observed in the change in CAR-T cell count (Fig. 9) and cytotoxic activity (Fig. 10) upon continuous antigen stimulation. In particular, CAR-T 2B, which had a linker length of 10 amino acids, showed no proliferation ability, and also no cytotoxic activity on day 6.

Subsequently, these CAR-T cell formulations were evaluated for drug efficacy in vivo (Fig. 11A). The evaluation method was the same as that described in Example 3; $1 \times 10^6$ CAR-T cells were intravenously injected into the tail at a dose of 0.1 mL/body once on the same day of grouping. Fig. 11B shows the efficacy of CAR-T cells over time due the change in linker length. The activity of CAR-T 2B was weak, indicating limitations due to 10 amino acids. CAR-T cells with linker lengths from 15 amino acids to 25 amino acids showed strong proliferation inhibition and led to a notable regression of tumors on Day 14. Although CAR-T cells with a linker length of 15 amino acids showed strong regression activity, there was a slight increase in tumor until day 7, indicating that greater linker lengths enhance antitumor activity (Fig. 11B).

These results indicate that for CD98 CAR-T cells to exert their drug efficacy, it is important to use a linker of 15 amino acids or more.

Example 5: Effect of Linker Length on CD98 CAR-T Cell Activity 2

Additionally, the effect of the linker length connecting scFv VL and scFv VH on the efficacy of CD98 CAR-T cells was evaluated. Table 6 shows the CAR-T cells produced according to the method described in Example 4 in order to evaluate the effects of linker length on CD98 CAR-T cells.

Table 6

| | scFv Order | Linker Connecting VH and VL | Hinge or Spacer | Transmembrane | Signaling Domain |
|---|---|---|---|---|---|
| CAR-T 2A | VH-VL | 218 Linker | CD28 | CD28 | CD28 - CD3$\zeta$ |
| CAR-T 3B<br>DNA: SEQ ID NO: 52<br>AA: SEQ ID NO: 111 | VH-VL | G4S$\times$4 (20 Amino Acids) | CD28 | CD28 | CD28 - CD3$\zeta$ |
| CAR-T 3C<br>DNA: SEQ ID NO: 53<br>AA: SEQ ID NO: 112 | VH-VL | G4S$\times$5 (25 Amino Acids) | CD28 | CD28 | CD28 - CD3$\zeta$ |

(continued)

| | scFv Order | Linker Connecting VH and VL | Hinge or Spacer | Transmembrane | Signaling Domain |
|---|---|---|---|---|---|
| CAR-T 3D DNA: SEQ ID NO: 54 AA: SEQ ID NO: 113 | VH-VL | G4S×6 (30 Amino Acids) SEQ ID NO: 33 | CD28 | CD28 | CD28 - CD3ζ |
| CAR-T 3E DNA: SEQ ID NO: 55 AA: SEQ ID NO: 114 | VH-VL | G4S×7 (35 Amino Acids) SEQ ID NO: 34 | CD28 | CD28 | CD28 - CD3ζ |
| CAR-T 3F DNA: SEQ ID NO: 56 AA: SEQ ID NO: 115 | VH-VL | G4S×8 (40 Amino Acids) SEQ ID NO: 35 | CD28 | CD28 | CD28 - CD3ζ |
| CAR-T 3G DNA: SEQ ID NO: 57 AA: SEQ ID NO: 116 | VH-VL | G4S×10 (50 Amino Acids) SEQ ID NO: 36 | CD28 | CD28 | CD28 - CD3ζ |

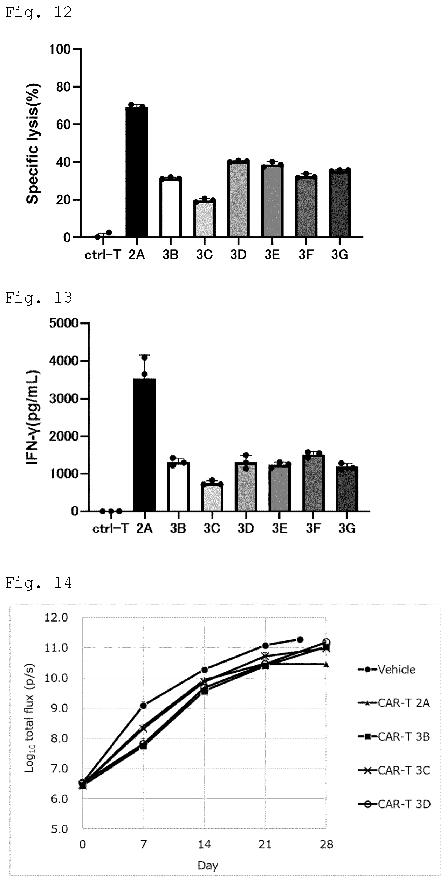

The results of this study indicated that compared with CAR-T 2A cells with a 218 linker, CAR-T 3B cells to CAR-T 3G cells with a linker length of 20 amino acids, 25 amino acids, 30 amino acids, 35 amino acids, 40 amino acids, and 50 amino acids exhibited reduced cytotoxic activity and reduced IFN-γ production ability (Figs. 12 and 13).

The in vivo efficacy of CAR-T cells with altered linker lengths was evaluated. The evaluation method was the same as that described in Example 3; $3 \times 10^5$ CAR-T cells were intravenously injected into the tail at a dose of 0.1 mL/body once on the same day of grouping.

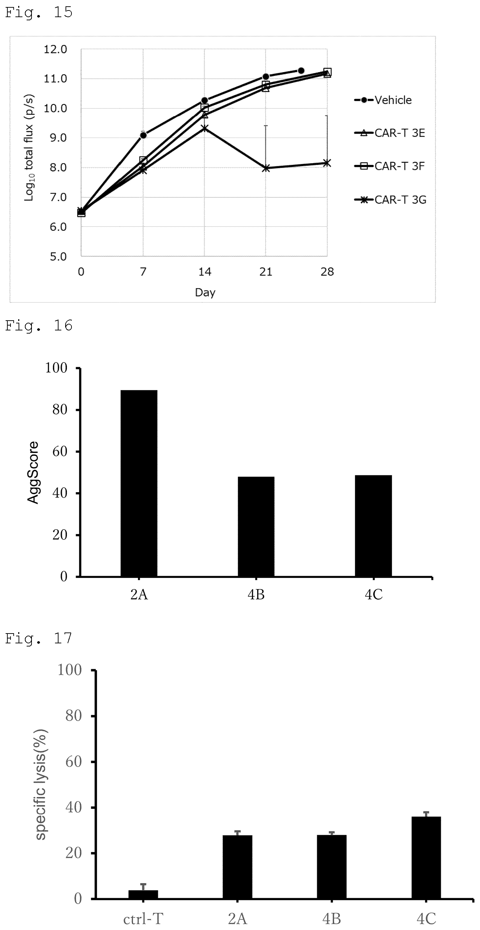

Figs. 14 and 15 show the efficacy of CAR-T cells due to altered linker lengths. CAR-T 3G cells slowly inhibited proliferation until day 14, followed by noticeable tumor regression afterward, showing antitumor activity as a tumor regression effect with a linker length of up to 50 amino acids (Fig. 15).

Example 6: Effect of CAR Aggregation on CD98 CAR-T Cell Activity

Assuming that CAR aggregation might affect the efficacy of CAR-T cells, Fv (fragment variable) structures with low aggregation were predicted by using BioLuminate (Schrödinger), and the efficacy of CAR-T cells produced based on these structures was evaluated.

In evaluating the degree of aggregation of CAR, the VH and VL sequences of CD98 CAR cells were input into Bioluminate, and the AggScore (aggregation evaluation score) for the predicted structures was calculated. Additionally, the residues affecting the increase in AggScore were identified, and the AggScore was also calculated for structures in which those residues were substituted with others.

The results of this study indicate that as compared with the structure before amino acid substitution ("CAR-T 2A" below), a decrease in AggScore was confirmed with a structure in which the 95th isoleucine in the VH sequence was substituted with arginine, the 12th proline in the VL sequence was substituted with arginine, and the 90th isoleucine in the VL sequence was substituted with arginine ("CAR-T 4B" below), and a structure in which the 95th isoleucine in the VH sequence was substituted with arginine, the 12th proline in the VL sequence was substituted with glutamine, and the 90th isoleucine in the VL sequence was substituted with arginine ("CAR-T 4C" below) (Fig. 16).

Table 7 shows the CAR-T cells produced according to the method described in Example 4 in order to evaluate the effects of aggregation in CAR structures on CD98 CAR-T cells.

Table 7

| | scFv Order | scFv Amino Acid Substitution | Linker Connecting VH and VL | Hinge or Spacer | Transmembrane | Signaling Domain |
|---|---|---|---|---|---|---|
| CAR-T 2A | VH-VL | - | 218 Linker | CD28 | CD28 | CD28 - CD3ζ |

(continued)

| | scFv Order | scFv Amino Acid Substitution | Linker Connecting VH and VL | Hinge or Spacer | Transmembrane | Signaling Domain |
|---|---|---|---|---|---|---|
| CAR-T 4B DNA: SEQ ID NO: 58 AA: SEQ ID NO: 117 | VH-VL | VH: I95R SEQ ID NO: 24 VL: P12R, I90R SEQ ID NO: 25 | 218 Linker | CD28 | CD28 | CD28 - CD3ζ |
| CAR-T 4C DNA: SEQ ID NO: 59 AA: SEQ ID NO: 118 | VH-VL | VH: I95R SEQ ID NO: 24 VL: P12Q, I90R SEQ ID NO: 27 | 218 Linker | CD28 | CD28 | CD28 - CD3ζ |

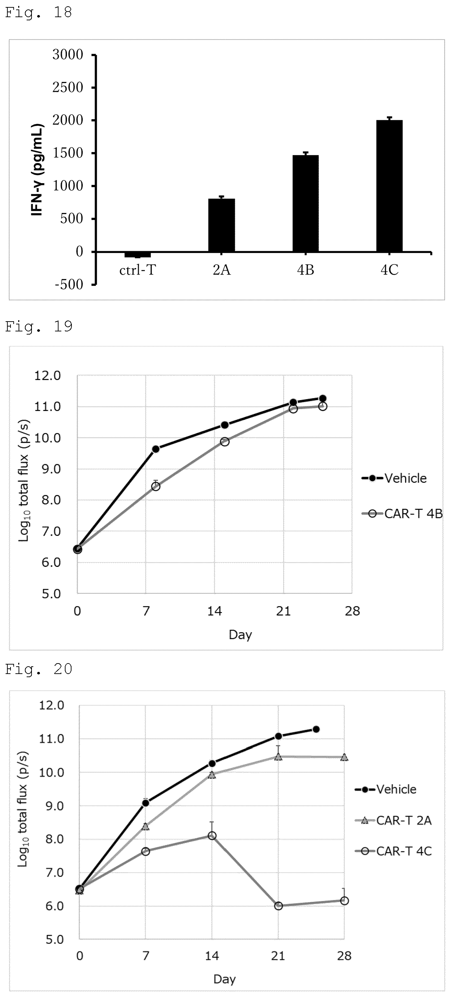

The results of this study indicate that compared with CAR-T 2A cells before amino acid substitution, CAR-T 4B cells and CAR-T 4C cells showed similar cytotoxic activity (Fig. 17). Both CAR-T 4B and CAR-T 4C cells exhibited improvement in IFN-γ production ability (Fig. 18).

The in vivo efficacy of CAR-T cells with substituted scFv amino acids was evaluated. The evaluation method was the same as that described in Example 3; $3 \times 10^5$ CAR-T cells were intravenously injected into the tail at a dose of 0.1 mL/body once on the same day of grouping.

Figs. 19 and 20 show the efficacy of CAR-T cells with substituted scFv amino acids. In the administration at a cell count of $3 \times 10^5$ CAR-T cells, CAR-T 4C cells showed proliferation inhibition up to day 14 compared with CAR-T 2A cells; after that, CAR-T 4C cells exhibited marked regression of tumor, showing a regression effect from the point in time of grouping (Fig. 20).

Example 7: Comparison of Efficacy between CD98 CAR-T Cells and CD19 CAR-T Cells

Efficacy was compared between CD98 CAR-T cells and CD19 CAR-T cells. Products such as KYMRIAH, YESCARTA, and Breyanzi (all product names), which are marketed as CAR-T cell therapies, are all CAR-T cells targeting CD19. To evaluate the effectiveness of CD98 CAR-T cells, their efficacy was compared with that of CD19 CAR-T cells.

Table 8 shows the CAR-T cells produced using lentiviral vectors in order to compare the efficacy between CD98 CAR-T cells (CAR-T 2A) and CD19 CAR-T cells (CAR-T 5B). CD19 CAR-T cells were produced using a lentiviral vector according to the CAR sequence of KYMRIAH.

Table 8

| | scFv Order | Linker Connecting VH and VL | Hinge or Spacer | Transmembrane | Signaling Domain |
|---|---|---|---|---|---|
| CD98 CAR-T (CAR-T 2A) | VH-VL | 218 Linker | CD28 | CD28 | CD28 - CD3ζ |
| CD19 CAR-T (CAR-T 5B) DNA: SEQ ID NO: 60 AA: SEQ ID NO: 119 | VL-VH | G4S×3(15 Amino Acids) | CD8α | CD8α | 4-1BB - CD3ζ |

The evaluation of CAR-T cells was performed according to the same method as that described in Example 2.

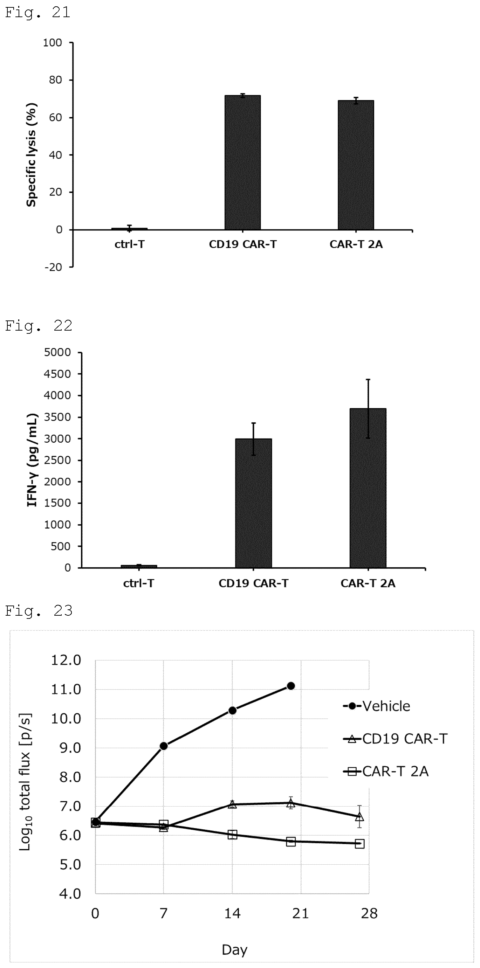

In comparison of in vitro efficacy between CAR-T 2A cells and CD19 CAR-T cells, these cells showed similar levels of cytotoxic activity (Fig. 21). There was also no significant difference in IFN-γ production ability (Fig. 22).

In vivo efficacy was compared between CAR-T 2A cells and CD19 CAR-T cells. The evaluation method was the same as that described in Example 3; $3 \times 10^6$ CAR-T cells were intravenously injected into the tail at a dose of 0.1 mL/body once on the same day of grouping.

In comparison of in vivo efficacy between CAR-T 2A cells and CD19 CAR-T cells, CAR-T 2A cells showed strong proliferation inhibition and tumor regression after administration. In contrast, the control CD19 CAR-T cells suppressed tumor growth but did not achieve notable regression. Thus, the difference between CAR-T 2A cells and CD19 CAR-T cells was significant (Fig. 23).

Example 8: In Vivo Evaluation for Other Cancer Types

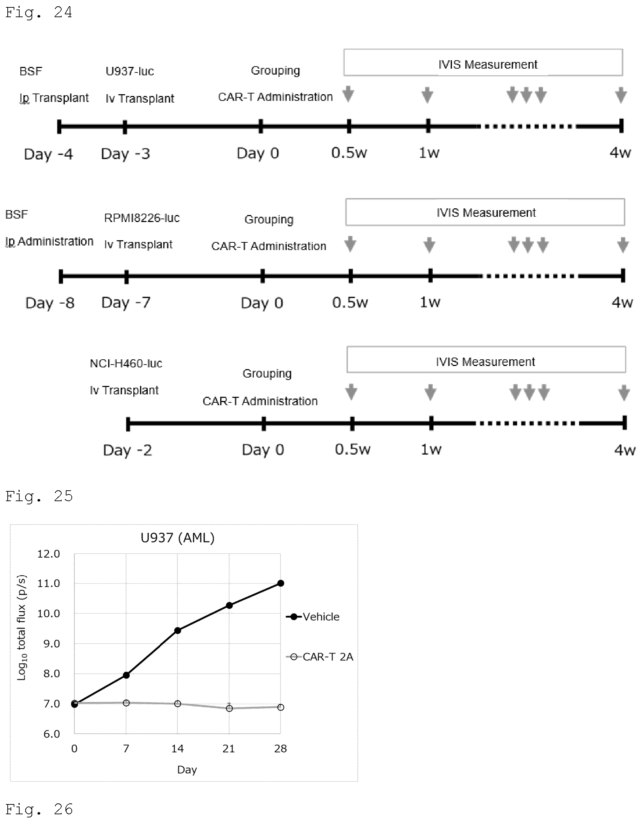

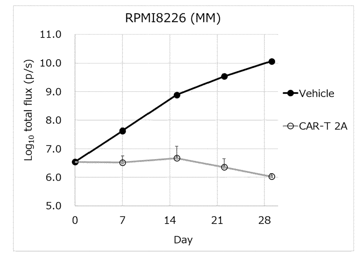

Antitumor effects against other types of cancer were evaluated using luciferase gene-transfected cell lines of three cancer types. In vivo efficacy of CAR-T 2A was evaluated according to the following procedure by using the following cells: U937-luc cells (ATCC, CRL-1593.2), which are an acute myelogenous leukemia (AML) cell line, and RPMI8226-luc cells (ATCC, CCL-155), which are a multiple myeloma (MM) cell line, for blood cancer; and NCI-H460-luc cells (ATCC, HTB-177), which are non-small-cell lung cancer (NSCLC), for solid cancer. NOD/Shi-scid, IL-2RγKO Jic (NOG) mice produced by CLEA Japan Inc. were delivered at 6 weeks of age. At 7 weeks of age, U937-luc cells ($1\times10^5$ cells/0.1 mL/body), RPMI8226-luc cells ($5\times10^5$ cells/0.1 mL/body), and NCI-H460-luc cells ($2\times10^5$ cells/0.1 mL/body) were transplanted via the tail vein to create tumor-bearing mice. In a blood cancer transplant, 20 mg/kg of Busulfex was administered intraperitoneally the day before transplantation. According to the schedule shown in Fig. 24 below, luminescence values were measured using an IVIS Lumina X5 after transplantation, and the luminescence values were used as an index to divide the mice into groups using a stratified random sampling method with SAS software R9.4. The CAR-T cells to be administered were $2\times10^6$ CAR-T cells for the AML model and MM model, and $3\times10^6$ CAR-T cells for the NSCLC model. The cells were intravenously administered into the tail at a dose of 0.1 mL/body once on the same day of grouping. The drug efficacy was evaluated based on the tumor luminescence values obtained with IVIS Lumina X5 as an index. 15 mg/mL VivoGlo-luciferin was intraperitoneally injected at a dose of 10 mL/kg based on the previously measured body weight to measure the luminescence value of the tumor. The total flux (p/s) of the tumor luminescence value means photons/second.

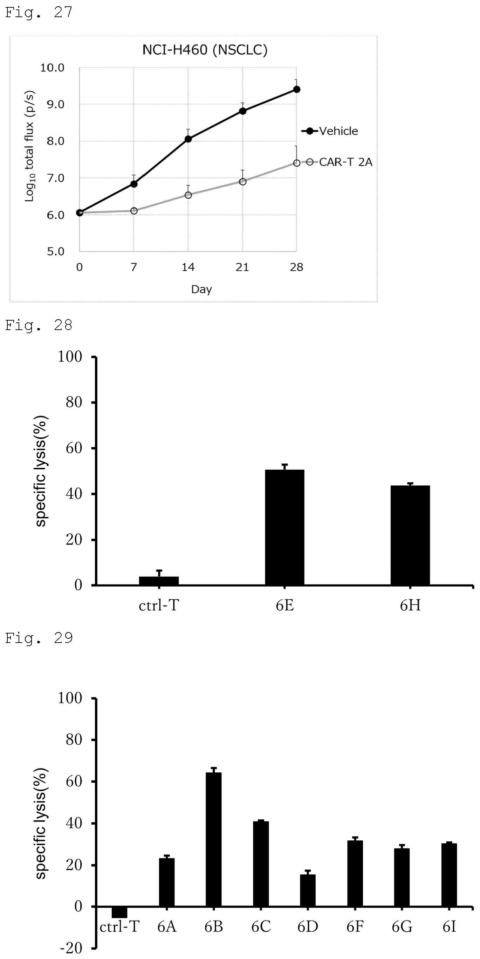

The antitumor effects on other types of cancer were the following: for blood cancer, complete proliferation inhibition effects on AML cells (Fig. 25) and regression effects on MM cells (Fig. 26) were confirmed; furthermore, effectiveness against NSCLC cells (solid tumor) was also confirmed (Fig. 27). Significant proliferation inhibition effects were observed in both types of cancer.

Example 9: Evaluation of Impact of Combination of Domains on CD98 CAR-T Cell Activity

CAR structures were prepared by combining a signaling domain with scFv sequences and linker length, which were found to affect CD98 CAR-T cell activity in previous studies, and the effect on CD98 CAR-T cell activity was evaluated.

Tables 9 to 13 show the CAR structures of CAR-T cells prepared according to the method described in Example 4 in order to evaluate the effects of various combinations of domains of CAR on CD98 CAR-T cells.

Table 9

| | scFv Order | scFv Amino Acid Substitution | Linker Connecting VH and VL | Hinge or Spacer | Transmembrane | Signaling Domain |
|---|---|---|---|---|---|---|
| CAR-T 6A<br>DNA: SEQ ID NO: 61<br>AA: SEQ ID NO: 120 | VH-VL | - | 218 Linker | CD28 | CD28 | CD28 - CD3ζ |
| CAR-T 6B<br>DNA: SEQ ID NO: 62<br>AA: SEQ ID NO: 121 | VH-VL | - | 218 Linker | CD8a | CD8a | 4-1BB - CD3ζ |
| CAR-T 6C<br>DNA: SEQ ID NO: 63<br>AA: SEQ ID NO: 122 | VH-VL | - | 218 Linker | CD8a | CD28 | CD28 - 4-1BB - CD3ζ |
| CAR-T 6D<br>DNA: SEQ ID NO: 64<br>AA: SEQ ID NO: 123 | VH-VL | - | G4S×4 (20 Amino Acids) | CD28 | CD28 | CD28 - CD3ζ |
| CAR-T 6E<br>DNA: SEQ ID NO: 65<br>AA: SEQ ID NO: 124 | VH-VL | - | G4S×4 (20 Amino Acids) | CD8a | CD8a | 4-1BB - CD3ζ |
| CAR-T 6F<br>DNA: SEQ ID NO: 66<br>AA: SEQ ID NO: 125 | VH-VL | - | G4S×4 (20 Amino Acids) | CD8a | CD28 | CD28 - 4-1BB - CD3ζ |

(continued)

| | scFv Order | scFv Amino Acid Substitution | Linker Connecting VH and VL | Hinge or Spacer | Transmembrane | Signaling Domain |
|---|---|---|---|---|---|---|
| CAR-T 6G DNA: SEQ ID NO: 67 AA: SEQ ID NO: 126 | VH-VL | - | G4S×5 (25 Amino Acids) | CD28 | CD28 | CD28 - CD3ζ |
| CAR-T 6H DNA: SEQ ID NO: 68 AA: SEQ ID NO: 127 | VH-VL | - | G4S×5 (25 Amino Acids) | CD8a | CD8a | 4-1BB - CD3ζ |
| CAR-T 6I DNA: SEQ ID NO: 69 AA: SEQ ID NO: 128 | VH-VL | - | G4S×5 (25 Amino Acids) | CD8a | CD28 | CD28 - 4-1BB - CD3ζ |

Table 10

| | scFv Order | scFv Amino Acid Substitution | Linker Connecting VH and VL | Hinge or Spacer | Transmembrane | Signaling Domain |
|---|---|---|---|---|---|---|
| CAR-T 7A DNA: SEQ ID NO: 70 AA: SEQ ID NO: 129 | VH-VL | - | G4S×10 (50 Amino Acids) | CD28 | CD28 | CD28 - CD3ζ |
| CAR-T 7B DNA: SEQ ID NO: 71 AA: SEQ ID NO: 130 | VH-VL | - | G4S×10 (50 Amino Acids) | CD8a | CD8a | 4-1BB - CD3ζ |
| CAR-T 7C DNA: SEQ ID NO: 72 AA: SEQ ID NO: 131 | VH-VL | - | G4S×10 (50 Amino Acids) | CD8a | CD28 | CD28 - 4-1BB - CD3ζ |
| CAR-T 7D DNA: SEQ ID NO: 73 AA: SEQ ID NO: 132 | VH-VL | VH: I95R VL: P12Q, I90R | 218 Linker | CD28 | CD28 | CD28 - CD3ζ |
| CAR-T 7E DNA: SEQ ID NO: 74 AA: SEQ ID NO: 133 | VH-VL | VH: I95R VL: P12Q, I90R | 218 Linker | CD8a | CD8a | 4-1BB - CD3ζ |
| CAR-T 7F DNA: SEQ ID NO: 75 AA: SEQ ID NO: 134 | VH-VL | VH: I95R VL: P12Q, I90R | 218 Linker | CD8a | CD28 | CD28 - 4-1BB - CD3ζ |
| CAR-T 7G DNA: SEQ ID NO: 76 AA: SEQ ID NO: 135 | VH-VL | VH: I95R VL: P12Q, I90R | G4S×5 (25 Amino Acids) | CD28 | CD28 | CD28 - CD3ζ |
| CAR-T 7H DNA: SEQ ID NO: 77 AA: SEQ ID NO: 136 | VH-VL | VH: I95R VL: P12Q, I90R | G4S×5 (25 Amino Acids) | CD8a | CD8a | 4-1BB - CD3ζ |
| CAR-T 7I DNA: SEQ ID NO: 78 AA: SEQ ID NO: 137 | VH-VL | VH: I95R VL: P12Q, I90R | G4S×5 (25 Amino Acids) | CD8a | CD28 | CD28 - 4-1BB - CD3ζ |

Table 11

| | scFv Order | scFv Amino Acid Substitution | Linker Connecting VH and VL | Hinge or Spacer | Transmembrane | Signaling Domain |
|---|---|---|---|---|---|---|
| CAR-T 8A DNA: SEQ ID NO: 79 AA: SEQ ID NO: 138 | VH-VL | - | G4S×6 (30 Amino Acids) | CD28 | CD28 | CD28 - CD3ζ |
| CAR-T 8B DNA: SEQ ID NO: 80 AA: SEQ ID NO: 139 | VH-VL | - | G4S×6 (30 Amino Acids) | CD8a | CD8a | 4-1BB - CD3ζ |
| CAR-T 8C DNA: SEQ ID NO: 81 AA: SEQ ID NO: 140 | VH-VL | - | G4S×6 (30 Amino Acids) | CD8a | CD28 | CD28 - 4-1BB - CD3ζ |
| CAR-T 8D DNA: SEQ ID NO: 82 AA: SEQ ID NO: 141 | VH-VL | VH: I95R VL: P12Q, I90R | G4S×6 (30 Amino Acids) | CD8a | CD8a | 4-1BB - CD3ζ |
| CAR-T 8E DNA: SEQ ID NO: 83 AA: SEQ ID NO: 142 | VH-VL | VH: I95R VL: P12Q, I90R | G4S×6 (30 Amino Acids) | CD8a | CD28 | CD28 - 4-1BB - CD3ζ |
| CAR-T 8F DNA: SEQ ID NO: 84 AA: SEQ ID NO: 143 | VH-VL | VH: I95R VL: P12Q, I90R | G4S×10 (50 Amino Acids) | CD28 | CD28 | CD28 - CD3ζ |
| CAR-T 8G DNA: SEQ ID NO: 83 AA: SEQ ID NO: 144 | VH-VL | VH: I95R VL: P12Q, I90R | G4S×10 (50 Amino Acids) | CD8a | CD8a | 4-1BB - CD3ζ |
| CAR-T 8H DNA: SEQ ID NO: 86 AA: SEQ ID NO: 145 | VH-VL | VH: I95R VL: P12Q, I90R | G4S×10 (50 Amino Acids) | CD8a | CD28 | CD28 - 4-1BB - CD3ζ |

Table 12

| | scFv Order | scFv Amino Acid Substitution | Linker Connecting VH and VL | Hinge or Spacer | Transmembrane | Signaling Domain |
|---|---|---|---|---|---|---|
| CAR-T 9A DNA: SEQ ID NO: 87 AA: SEQ ID NO: 146 | VH-VL | VH: I95R VL: P12Q, I90R | G4S×6 (30 Amino Acids) | CD28 | CD28 | CD28 - CD3ζ |
| CAR-T 9B DNA: SEQ ID NO: 88 AA: SEQ ID NO: 147 | VH-VL | VH: I95R VL:P12R, I90R | 218 Linker | CD28 | CD28 | CD28 - CD3ζ |
| CAR-T 9C DNA: SEQ ID NO: 89 AA: SEQ ID NO: 148 | VH-VL | VH: I95R VL: P12R, I90R | 218 Linker | CD8a | CD8a | 4-1BB - CD3ζ |
| CAR-T 9D DNA: SEQ ID NO: 90 AA: SEQ ID NO: 149 | VH-VL | VH: I95R VL: P12R, I90R | 218 Linker | CD8a | CD28 | CD28 - 4-1BB - CD3ζ |
| CAR-T 9E DNA: SEQ ID NO: 91 AA: SEQ ID NO: 150 | VH-VL | VH: I95R VL: P12R, I90R | G4S×5 (25 Amino Acids) | CD28 | CD28 | CD28 - CD3ζ |

(continued)

| | scFv Order | scFv Amino Acid Substitution | Linker Connecting VH and VL | Hinge or Spacer | Transmembrane | Signaling Domain |
|---|---|---|---|---|---|---|
| CAR-T 9F DNA: SEQ ID NO: 92 AA: SEQ ID NO: 151 | VH-VL | VH: I95R VL: P12R, I90R | G4Sx5 (25 Amino Acids) | CD8a | CD8a | 4-1BB - CD3ζ |
| CAR-T 9G DNA: SEQ ID NO: 93 AA: SEQ ID NO: 152 | VH-VL | VH: I95R VL: P12R, I90R | G4S×5 (25 Amino Acids) | CD8a | CD28 | CD28 - 4-1BB - CD3ζ |
| CAR-T 9H DNA: SEQ ID NO: 94 AA: SEQ ID NO: 153 | VH-VL | VH: I95R VL: P12R, I90R | G4S×6 (30 Amino Acids) | CD28 | CD28 | CD28 - CD3ζ |
| CAR-T 9I DNA: SEQ ID NO: 95 AA: SEQ ID NO: 154 | VH-VL | VH: I95R VL: P12R, I90R | G4S×6 (30 Amino Acids) | CD8a | CD8a | 4-1BB - CD3ζ |
| CAR-T 9J DNA: SEQ ID NO: 96 AA: SEQ ID NO: 155 | VH-VL | VH: I95R VL: P12R, I90R | G4S×6 (30 Amino Acids) | CD8a | CD28 | CD28 - 4-1BB - CD3ζ |

(Continued from Table 12)

| | scFv Order | scFv Amino Acid Substitution | Linker Connecting VH and VL | Hinge or Spacer | Transmembrane | Signaling Domain |
|---|---|---|---|---|---|---|
| CAR-T 9K DNA: SEQ ID NO: 97 AA: SEQ ID NO: 156 | VH-VL | VH: I95R VL: P12R, I90R | G4S×10 (50 Amino Acids) | CD28 | CD28 | CD28 - CD3ζ |
| CAR-T 9L DNA: SEQ ID NO: 98 AA: SEQ ID NO: 157 | VH-VL | VH: I95R VL: P12R, I90R | G4S×10 (50 Amino Acids) | CD8α | CD8α | 4-1BB - CD3ζ |
| CAR-T 9M DNA: SEQ ID NO: 99 AA: SEQ ID NO: 158 | VH-VL | VH: I95R VL: P12R, I90R | G4S×10 (50 Amino Acids) | CD8α | CD28 | CD28 - 4-1BB - CD3ζ |

Table 13

| | scFv Order | scFv Amino Acid Substitution | Linker Connecting VH and VL | Hinge or Spacer | Transmembrane | Signaling Domain |
|---|---|---|---|---|---|---|
| CAR-T 10A DNA: SEQ ID NO: 100 AA: SEQ ID NO: 159 | VH-VL | VH: I95R VL: P12Q, I90R | 218 Linker | CD28 | CD28 | CD28 - CD3ζ |
| CAR-T 10B DNA: SEQ ID NO: 101 AA: SEQ ID NO: 160 | VH-VL | VH: I95R VL: P12Q, I90R | 218 Linker | CD8α | CD8α | 4-1BB - CD3ζ |
| CAR-T 10C DNA: SEQ ID NO: 102 AA: SEQ ID NO: 161 | VH-VL | VH: I95R VL: P12Q, I90R | G4S×5 (25 Amino Acids) | CD8α | CD8α | 4-1BB - CD3ζ |

(continued)

| | scFv Order | scFv Amino Acid Substitution | Linker Connecting VH and VL | Hinge or Spacer | Transmembrane | Signaling Domain |
|---|---|---|---|---|---|---|
| CAR-T 10D DNA: SEQ ID NO: 103 AA: SEQ ID NO: 162 | VH-VL | VH: I95R VL: P12Q, I90R | G4S×10 (50 Amino Acids) | CD8α | CD8α | 4-1BB - CD3ζ |

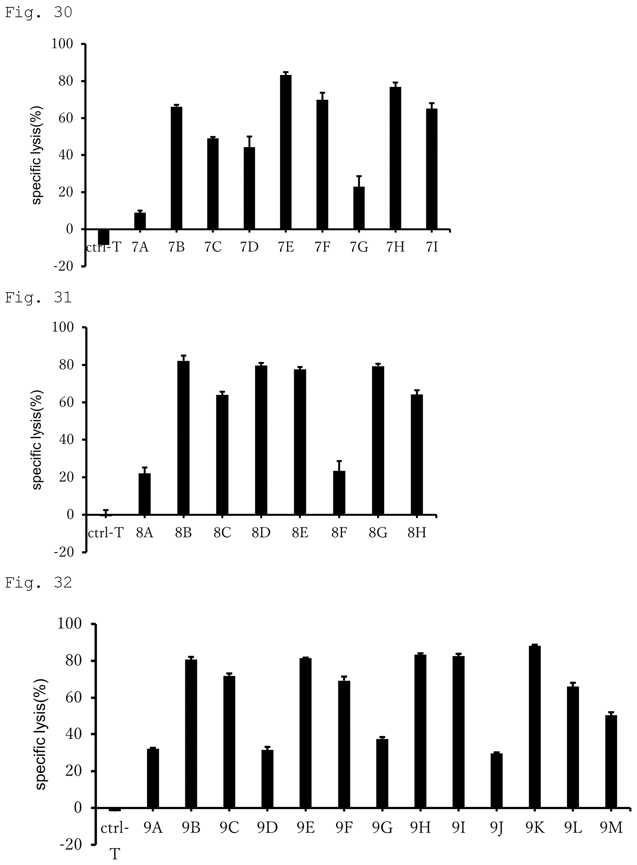

The results of this study indicated that the constructs in the test of Table 9 all showed cytotoxic activity as compared with the control; in particular, constructs other than those with a structure composed of a combination of a hinge or spacer derived from CD28, a transmembrane derived from CD28, and a signaling domain of CD28-CD3ζ showed further improved activity (Figs. 28 and 29). This trend was also observed with amino acid substitutions of scFv in the combinations shown in Tables 10, 11, 12, and 13 (Figs. 30, 31, 32, and 33). Among the structures compared for cytotoxicity simultaneously, no significant difference in cytotoxic activity was observed between the structures in which the regions downstream of the hinge were equivalent.

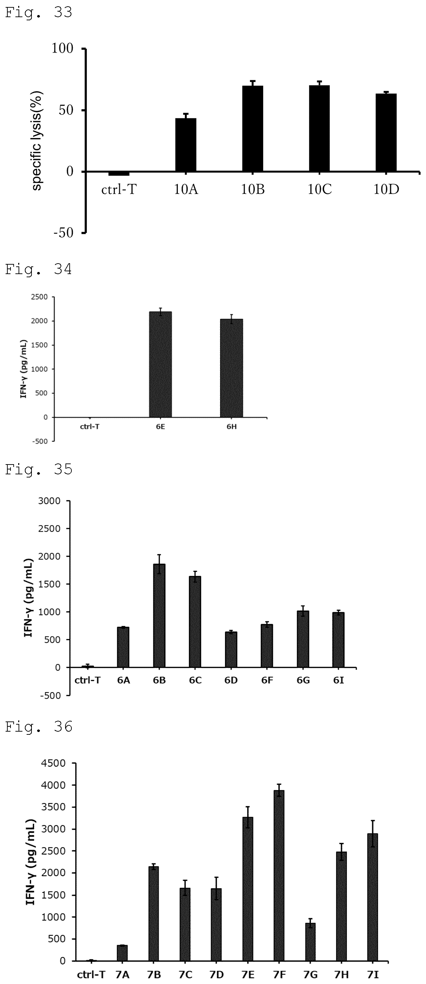

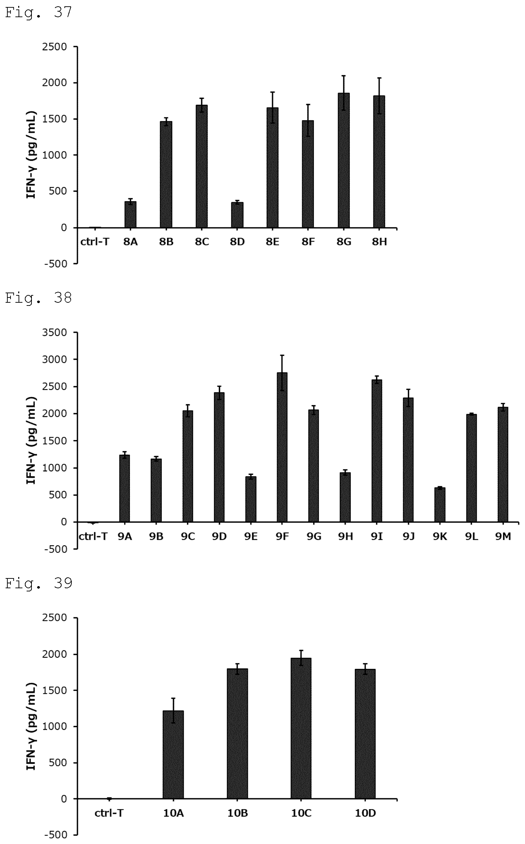

The results of evaluating the IFN-γ production ability indicated that the amount of IFN-γ produced was increased in all constructs compared with the control. In particular, compared with the constructs with a structure in which the hinge or spacer was derived from CD28, the transmembrane was derived from CD28, and the signaling domain was CD28-CD3ζ, the constructs with structures other than this combination had a tendency to produce a higher amount of IFN-γ (Figs. 34, 35, 36, 37, 38, and 39).

The in vivo efficacy of CAR-T cells of various combinations shown in Tables 9, 10, 11, 12, and 13 was evaluated. The evaluation method was the same as that described in Example 3; $3 \times 10^5$ CAR-T cells were intravenously injected into the tail at a dose of 0.1 mL/body once on the same day of grouping.

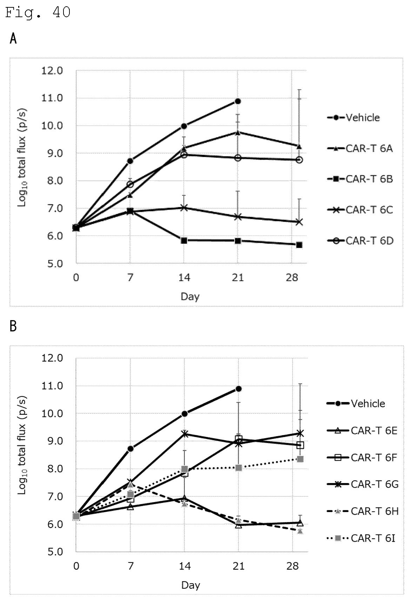

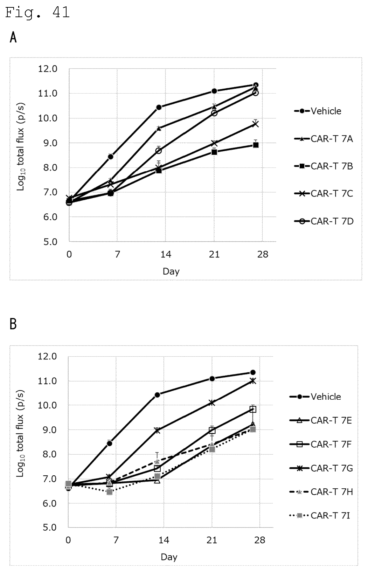

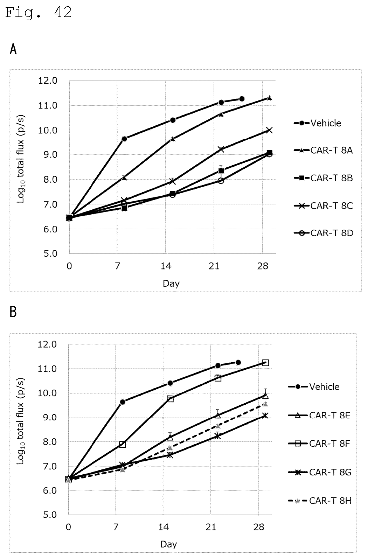

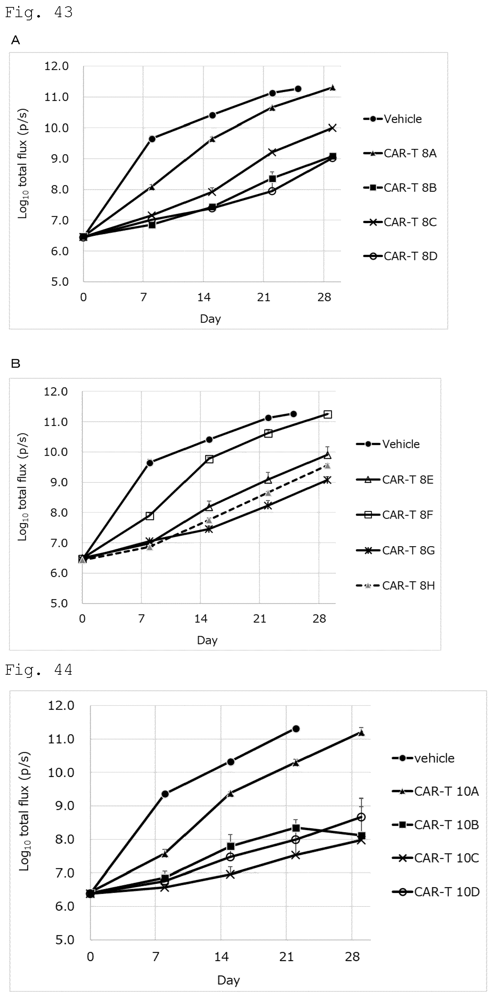

Figs. 40, 41, 42, 43, and 44 show the in vivo efficacy of CAR-T cells of various combinations shown in Tables 9, 10, 11, 12, and 13. All combinations showed proliferation inhibition compared with the vehicle. In particular, CAR-T 6B, CAR-T 6C, CAR-T 6E, and CAR-T 6H showed a significant tumor growth inhibition effect, and all showed a strong regression effect on day 14 (Fig. 40).

## Example 10: Effect of Dasatinib on CD98 CAR-T Cell Activity

The effect of dasatinib, which was added during CAR-T cell generation, on the efficacy of CD98 CAR-T cells was evaluated.

In order to evaluate the effects of dasatinib on CD98 CAR-T cells, dasatinib-added CAR-T cells and unadded CAR-T cells were produced according to the method described in Example 4. Table 14 shows the produced CAR-T cells.

Table 14

| | scFv Order | scFv Amino Acid Substitution | Linker Connecting VH and VL | Hinge or Spacer | Transmembrane | Signaling Domain | Dasatinib |
|---|---|---|---|---|---|---|---|
| CAR-T 2A | VH-VL | - | 218 Linker | CD28 | CD28 | CD28 - CD3ζ | + |
| CAR-T 11A | VH-VL | - | 218 Linker | CD28 | CD28 | CD28 - CD3ζ | - |

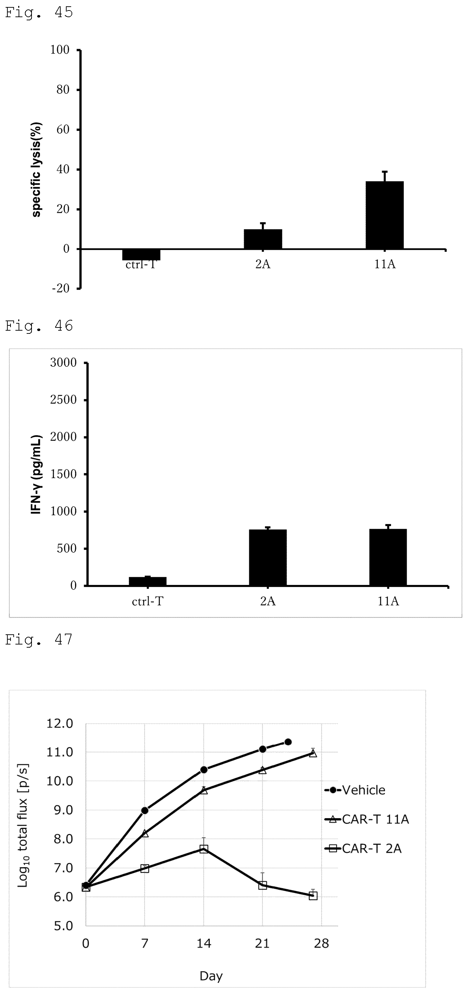

The results of this study indicated that compared with CAR-T 2A cells generated with dasatinib, CAR-T 11A cells generated without dasatinib exhibited improvement in cytotoxic activity (Fig. 45). CAR-T 2A cells and CAR-T 11A cells both showed similar IFN-γ production ability (Fig. 46).

The in vivo efficacy of dasatinib was compared in CAR-T 2A and CAR-T 11A. The evaluation method was the same as that described in Example 3; $1 \times 10^6$ CAR-T cells were intravenously injected into the tail at a dose of 0.1 mL/body once on the same day of grouping.

In the in vivo efficacy study of CAR-T 2A and CAR-T 11A, CAR-T 2A generated with dasatinib showed significant growth inhibition until day 14 compared to CAR-T 11A generated without dasatinib. Subsequently, CAR-T 2A showed a significant antitumor effect until day 28, around which the tumor volume decreased to below the level observed at the start of CAR-T cell administration (Fig. 47).

## Claims

1. A chimeric antigen receptor comprising an scFv region having a structure in which the C-terminus of a heavy-chain variable region and the N-terminus of a light-chain variable region are linked via a linker, wherein

    the heavy-chain variable region contains

        a heavy-chain CDR1 having the amino acid sequence of SEQ ID NO: 1,
        a heavy-chain CDR2 having the amino acid sequence of SEQ ID NO: 2, and/or
        a heavy-chain CDR3 having the amino acid sequence of SEQ ID NO: 3, and/or

    the light-chain variable region contains

        a light-chain CDR1 having the amino acid sequence of SEQ ID NO: 5,
        a light-chain CDR2 having the amino acid sequence of SEQ ID NO: 6, and/or
        a light-chain CDR3 having the amino acid sequence of SEQ ID NO: 7; and

    the number of amino acid residues constituting the linker is 15 to 50.

2. The chimeric antigen receptor according to claim 1, wherein

    the amino acid sequence of a heavy-chain FR region of the heavy-chain variable region contains an amino acid sequence having at least 80% identity to the amino acid sequence of a heavy-chain FR region of the amino acid sequence of SEQ ID NO: 4, and/or
    the amino acid sequence of a light-chain FR region of the light-chain variable region contains an amino acid sequence having at least 80% identity to the amino acid sequence of a light-chain FR region of the amino acid sequence of SEQ ID NO: 8.

3. A chimeric antigen receptor, comprising

    a heavy-chain variable region,
    a light-chain variable region, and
    a linker,

    wherein

    the amino acid sequence of the heavy-chain variable region contains an amino acid sequence having at least 90% identity to the amino acid sequence of SEQ ID NO: 4, and/or
    the amino acid sequence of the light-chain variable region contains an amino acid sequence having at least 90% identity to the amino acid sequence of SEQ ID NO: 8; and
    the number of amino acid residues constituting the linker is 15 to 50.

4. The chimeric antigen receptor according to any one of claims 1 and 3, having a structure in which the heavy-chain variable region, the linker, the light-chain variable region, a hinge sequence, a transmembrane domain, and an intracellular signaling domain are arranged in this order from the N-terminus.

5. A polynucleotide encoding the chimeric antigen receptor of any one of claims 1 to 4.

6. A cell comprising the polynucleotide of claim 5.

7. The cell according to claim 6, which is a chimeric antigen receptor T cell.

8. A pharmaceutical composition comprising the cell of claim 6 or 7.

9. The pharmaceutical composition according to claim 8, which is for use in the treatment and/or prevention of cancer.

Fig. 1

LTR
VH
linker
VL
CD28
Hinge/Spacer
CD28
transmembrane
CD28-CD3ζ
Signal domain
LTR
CD98 scFv
LTR
VL
linker
VH
CD28
Hinge/Spacer
CD28
transmembrane
CD28-CD3ζ
Signal domain
LTR

Fig. 2

200
150
100
50
0
proliferation rat
(vs Day2)
ctrl-T
1A
1B

Fig. 3

100
80
60
40
20
0
specific lysis(%)
ctrl-T
1A
1B

Fig. 4

8000
6000
4000
2000
0
IFN-γ (pg/mL)
ctrl-T
1A
1B

Fig. 5

Day 7
1.0E+10
1.0E+09
1.0E+08
1.0E+07
1.0E+06
1.0E+05
1.0E+04
Photons/sec
Vehicle
1A
1B

Fig. 6

40
30
20
10
0
Proliferation
ctrl-T
2A
2B
2C
2D
2E

Fig. 7

Specific lysis(%)
100
80
60
40
20
0
ctrl-T
2A
2B
2C
2D
2E

Fig. 8

IFN-γ(pg/mL)
3000
2000
1000
0
ctrl-T
2A
2B
2C
2D
2E

Fig. 9

Total cell count
1000
100
10
1
0.1
0
3
6
10
13
17
days following initial stimulation
2A
2B
2C
2D
2E
Antigen stimulation

Fig. 10

Specific lysis(%)
100
80
60
40
20
0
0
3
6
10
13
17
days following initial stimulation
2A
2B
2C
2D
2E
Antigen stimulation

Fig. 11

A

Day 7
Photons/sec
1.0E+10
1.0E+09
1.0E+08
1.0E+07
1.0E+06
1.0E+05
1.0E+04
Vehicle
2A
2B
2C
2D
2E

B

$Log_{10}$ total flux [p/s]
12.0
11.0
10.0
9.0
8.0
7.0
6.0
5.0
0
7
14
21
28
Day
Vehicle
CAR-T 2A
CAR-T 2B
CAR-T 2C
CAR-T 2D
CAR-T 2E

Fig. 12

Specific lysis(%)
100
80
60
40
20
0
ctrl-T
2A
3B
3C
3D
3E
3F
3G

Fig. 13

IFN-γ(pg/mL)
5000
4000
3000
2000
1000
0
ctrl-T
2A
3B
3C
3D
3E
3F
3G

Fig. 14

Log10 total flux (p/s)
12.0
11.0
10.0
9.0
8.0
7.0
6.0
5.0
0
7
14
21
28
Day
Vehicle
CAR-T 2A
CAR-T 3B
CAR-T 3C
CAR-T 3D

Fig. 15

12.0
11.0
10.0
9.0
8.0
7.0
6.0
5.0
Log10 total flux (p/s)
0
7
14
21
28
Day
Vehicle
CAR-T 3E
CAR-T 3F
CAR-T 3G

Fig. 16

100
80
60
40
20
0
AggScore
2A
4B
4C

Fig. 17

100
80
60
40
20
0
specific lysis(%)
ctrl-T
2A
4B
4C

Fig. 18

IFN-γ (pg/mL)
3000
2500
2000
1500
1000
500
0
-500
ctrl-T
2A
4B
4C


Fig. 19

Log10 total flux (p/s)
12.0
11.0
10.0
9.0
8.0
7.0
6.0
5.0
0
7
14
21
28
Day
Vehicle
CAR-T 4B


Fig. 20

Log10 total flux (p/s)
12.0
11.0
10.0
9.0
8.0
7.0
6.0
5.0
0
7
14
21
28
Day
Vehicle
CAR-T 2A
CAR-T 4C

Fig. 21

Specific lysis (%)
100
80
60
40
20
0
-20
ctrl-T
CD19 CAR-T
CAR-T 2A

Fig. 22

IFN-γ (pg/mL)
5000
4500
4000
3500
3000
2500
2000
1500
1000
500
0
ctrl-T
CD19 CAR-T
CAR-T 2A

Fig. 23

Log10 total flux [p/s]
12.0
11.0
10.0
9.0
8.0
7.0
6.0
5.0
4.0
0
7
14
21
28
Day
Vehicle
CD19 CAR-T
CAR-T 2A

Fig. 24

BSF
Ip Transplant
U937-luc
Iv Transplant
Grouping
CAR-T Administration
IVIS Measurement
Day -4
Day -3
Day 0
0.5w
1w
4w
BSF
Ip Administration
RPMI8226-luc
Iv Transplant
Grouping
CAR-T Administration
IVIS Measurement
Day -8
Day -7
Day 0
0.5w
1w
4w
NCI-H460-luc
Iv Transplant
Grouping
CAR-T Administration
IVIS Measurement
Day -2
Day 0
0.5w
1w
4w

Fig. 25

U937 (AML)
Log10 total flux (p/s)
12.0
11.0
10.0
9.0
8.0
7.0
6.0
0
7
14
21
28
Day
Vehicle
CAR-T 2A

Fig. 26

RPMI8226 (MM)
11.0
10.0
9.0
8.0
7.0
6.0
5.0
Log10 total flux (p/s)
0
7
14
21
28
Day
Vehicle
CAR-T 2A

Fig. 27

NCI-H460 (NSCLC)
$Log_{10}$ total flux (p/s)
10.0
9.0
8.0
7.0
6.0
5.0
0
7
14
21
28
Day
Vehicle
CAR-T 2A

Fig. 28

specific lysis(%)
100
80
60
40
20
0
ctrl-T
6E
6H

Fig. 29

specific lysis(%)
100
80
60
40
20
0
-20
ctrl-T
6A
6B
6C
6D
6F
6G
6I

Fig. 30

specific lysis(%)
100
80
60
40
20
0
-20
ctrl-T
7A
7B
7C
7D
7E
7F
7G
7H
7I

Fig. 31

specific lysis(%)
100
80
60
40
20
0
-20
ctrl-T
8A
8B
8C
8D
8E
8F
8G
8H

Fig. 32

specific lysis(%)
100
80
60
40
20
0
-20
ctrl-T
9A
9B
9C
9D
9E
9F
9G
9H
9I
9J
9K
9L
9M

Fig. 33

100
50
0
-50
specific lysis(%)
ctrl-T
10A
10B
10C
10D

Fig. 34

2500
2000
1500
1000
500
0
-500
IFN-γ (pg/mL)
ctrl-T
6E
6H

Fig. 35

3000
2500
2000
1500
1000
500
0
-500
IFN-γ (pg/mL)
ctrl-T
6A
6B
6C
6D
6F
6G
6I

Fig. 36

4500
4000
3500
3000
2500
2000
1500
1000
500
0
IFN-γ (pg/mL)
ctrl-T
7A
7B
7C
7D
7E
7F
7G
7H
7I

Fig. 37

IFN-γ (pg/mL)
2500
2000
1500
1000
500
0
-500
ctrl-T
8A
8B
8C
8D
8E
8F
8G
8H

Fig. 38

IFN-γ (pg/mL)
3500
3000
2500
2000
1500
1000
500
0
-500
ctrl-T
9A
9B
9C
9D
9E
9F
9G
9H
9I
9J
9K
9L
9M

Fig. 39

IFN-γ (pg/mL)
2500
2000
1500
1000
500
0
-500
ctrl-T
10A
10B
10C
10D

Fig. 40

A

12.0
11.0
10.0
9.0
8.0
7.0
6.0
5.0
Log10 total flux (p/s)
0
7
14
21
28
Day
Vehicle
CAR-T 6A
CAR-T 6B
CAR-T 6C
CAR-T 6D

B

12.0
11.0
10.0
9.0
8.0
7.0
6.0
5.0
Log10 total flux (p/s)
0
7
14
21
28
Day
Vehicle
CAR-T 6E
CAR-T 6F
CAR-T 6G
CAR-T 6H
CAR-T 6I

Fig. 41

A

12.0
11.0
10.0
9.0
8.0
7.0
6.0
5.0
Log10 total flux (p/s)
0
7
14
21
28
Day
Vehicle
CAR-T 7A
CAR-T 7B
CAR-T 7C
CAR-T 7D

B

12.0
11.0
10.0
9.0
8.0
7.0
6.0
5.0
Log10 total flux (p/s)
0
7
14
21
28
Day
Vehicle
CAR-T 7E
CAR-T 7F
CAR-T 7G
CAR-T 7H
CAR-T 7I

Fig. 42

A

12.0
11.0
10.0
9.0
8.0
7.0
6.0
5.0
Log10 total flux (p/s)
0
7
14
21
28
Day
Vehicle
CAR-T 8A
CAR-T 8B
CAR-T 8C
CAR-T 8D


B

12.0
11.0
10.0
9.0
8.0
7.0
6.0
5.0
Log10 total flux (p/s)
0
7
14
21
28
Day
Vehicle
CAR-T 8E
CAR-T 8F
CAR-T 8G
CAR-T 8H

Fig. 43

A

12.0
11.0
10.0
9.0
8.0
7.0
6.0
5.0
Log10 total flux (p/s)
0
7
14
21
28
Day
Vehicle
CAR-T 8A
CAR-T 8B
CAR-T 8C
CAR-T 8D

B

12.0
11.0
10.0
9.0
8.0
7.0
6.0
5.0
Log10 total flux (p/s)
0
7
14
21
28
Day
Vehicle
CAR-T 8E
CAR-T 8F
CAR-T 8G
CAR-T 8H

Fig. 44

12.0
11.0
10.0
9.0
8.0
7.0
6.0
5.0
Log10 total flux (p/s)
0
7
14
21
28
Day
vehicle
CAR-T 10A
CAR-T 10B
CAR-T 10C
CAR-T 10D

Fig. 45

specific lysis(%)
100
80
60
40
20
0
-20
ctrl-T
2A
11A

Fig. 46

IFN-γ (pg/mL)
3000
2500
2000
1500
1000
500
0
ctrl-T
2A
11A

Fig. 47

Log10 total flux [p/s]
12.0
11.0
10.0
9.0
8.0
7.0
6.0
5.0
0
7
14
21
28
Day
Vehicle
CAR-T 11A
CAR-T 2A

**INTERNATIONAL SEARCH REPORT**

International application No.
**PCT/JP2024/040523**

**A. CLASSIFICATION OF SUBJECT MATTER**

***C07K 19/00***(2006.01)i; ***A61K 35/17***(2025.01)i; ***A61P 35/00***(2006.01)i; ***C07K 14/705***(2006.01)i; ***C07K 16/30***(2006.01)i; ***C12N 5/10***(2006.01)i; ***C12N 5/0783***(2010.01)i; ***C12N 15/12***(2006.01)i; ***C12N 15/13***(2006.01)i; ***C12N 15/62***(2006.01)i
FI: C07K19/00; C07K14/705; C12N15/13; C12N5/10; C12N5/0783; A61P35/00; A61K35/17; C12N15/62 Z; C07K16/30 ZNA; C12N15/12

According to International Patent Classification (IPC) or to both national classification and IPC

**B. FIELDS SEARCHED**

Minimum documentation searched (classification system followed by classification symbols)

C07K1/00-19/00; C12N15/00-15/90; A61K35/17; A61P35/00;C12N5/10; C12N5/0783

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII), CAplus/REGISTRY (STN), UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | WO 2017/026497 A1 (OSAKA UNIVERSITY) 16 February 2017 (2017-02-16) paragraphs [0041], [0094], [0097]-[0099], [0105]-[0106], [0110]-[0111] | 1-9 |
| X | CN 110229236 A (FIRST AFFILIATED HOSPITAL ZHENGZHOU UNIVERSITY) 13 September 2019 (2019-09-13) claims 1-3, examples 1-3, fig. 1 | 1-9 |
| X | WO 2020/183158 A1 (LEUCID BIO LTD.) 17 September 2020 (2020-09-17) claims 1, 12-27, 33, 36, 47-63, 72, paragraphs [0202]-[0209], [0218]-[0221] | 1-9 |
| X | JP 2022-525318 A (RUTGERS, THE STATE UNIVERSITY OF NEW JERSEY) 12 May 2022 (2022-05-12) claims 1, 9-10, 20, 22, 36, 45 | 1-9 |

☐ Further documents are listed in the continuation of Box C. ☑ See patent family annex.

* Special categories of cited documents:
“A” document defining the general state of the art which is not considered to be of particular relevance
“D” document cited by the applicant in the international application
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **21 January 2025** | **28 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/040523**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/040523**

| Patent document cited in search report | Publication date (day/month/year) | Patent family member(s) | Publication date (day/month/year) |
|---|---|---|---|
| WO 2017/026497 A1 | 16 February 2017 | US 2018/0230212 A1<br>paragraphs [0153], [0203], [0214]-[0216], [0222]-[0223], [0227]-[0228]<br>EP 3336185 A1<br>CN 108138172 A | |
| CN 110229236 A | 13 September 2019 | (Family: none) | |
| WO 2020/183158 A1 | 17 September 2020 | US 2022/0152103 A1<br>EP 3937974 A1<br>CN 113811327 A | |
| JP 2022-525318 A | 12 May 2022 | WO 2020/190483 A1<br>claims 1, 9-10, 20, 22, 36, 45<br>US 2022/0152106 A1<br>EP 3938402 A1<br>CN 113853390 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017026497 A


### Non-patent literature cited in the description


- *J Immunol.*, 01 February 2011, vol. 186 (3), 1840-1848
- *J Immunol.*, 01 November 2009, vol. 183 (9), 5563-74
- *N Engl J Med.*, 16 October 2014, vol. 371 (16), 1507-17
- *Nat Med.*, 2017, vol. 23, 1436-1443